# EUROPEAN PATENT APPLICATION

(11) **EP 3 821 824 A1**
(43) Date of publication of application: **19.05.2021**
(21) Application number: 19835134.8
(22) Date of filing: 11.07.2019
(51) Int. Cl.: A61B 17/12

(54) **LIGATION DEVICE**

(30) Priority: 11.07.2018 WO PCT/JP2018/026229
(71) Applicant: ASAHI INTECC CO., LTD., Seto-shi, Aichi 489-0071 (JP)
(72) Inventor: KIKUCHI, Daisuke, Tokyo 152-0021 (JP); TSUKAMOTO, Toshihiko, Seto-shi, Aichi 489-0071 (JP); NARITA, Toshiyuki, Tokyo 160-0023 (JP); TAKAYANAGI, Katsuyoshi, Seto-shi, Aichi 489-0071 (JP); NAMIMA, Satoshi, Seto-shi Aichi 489-0071 (JP); ASAHATA, Erika, Seto-shi, Aichi 489-0071 (JP); UTANI, Takayuki, Seto-shi, Aichi 489-0071 (JP)
(74) Representative: Winter, Brandl - Partnerschaft mbB
(86) International application number: PCT/JP2019/027544
(87) International publication number: WO 2020/013287

(57) **Abstract**

The present invention provides a ligation device that can always secure a good endoscopic visual field.

A ligation device 1 includes a cylindrical inner cylinder 20 having a distal end portion 23; a slider 40 having a cylindrical shape, attached around the inner cylinder 20, movable with respect to the inner cylinder 20 along an axial direction of the inner cylinder 20, and having annular grooves 44a, 44b and 44c extending along a circumferential direction on an inner peripheral face 43; and ligation rings 60, 61 and 62 attached around the inner cylinder 20 and located in the annular grooves 44a, 44b and 44c.

## Description

### TECHNICAL FIELD

The present disclosure relates to a ligation device.

### BACKGROUND ART

A ligation device for ligating an affected area such as a diverticula and a varix formed in a patient's digestive tract or the like is known. Such a ligation device is disclosed in e.g. Patent Literature 1. With a ligation kit disclosed in Patent Literature 1, a slide cylinder is driven by a fluid, and an O-ring attached on an inner cylinder is pushed out and ejected from the inner cylinder by the slide cylinder to ligate an affected area.

### CITATION LIST

### Patent Literature

Patent Literature 1: Japanese Patent Laid-Open No. 7-59786

### SUMMARY OF INVENTION

### TECHNICAL PROBLEM

However, with the ligation kit in Patent Literature 1, the O-ring stretched and attached to the inner cylinder is pushed out by the fluid-driven slide cylinder to eject the O-ring from the inner cylinder. Thus, the O-ring falls under an endoscopic visual field, and when searching another affected area, a good visual field cannot be secured.

An object of the present disclosure is to provide a ligation device that can always secure a good endoscopic visual field.

### SOLUTION TO PROBLEM

In order to achieve this object, a ligation device according to a disclosed aspect includes a cylindrical inner cylinder having a distal end portion, a slider having a cylindrical shape, attached around the inner cylinder, movable with respect to the inner cylinder along an axial direction of the inner cylinder, and having an annular groove extending along a circumferential direction on an inner peripheral face, and a ligation ring attached around the inner cylinder and located in the annular groove. The inner cylinder may be located on e.g. a distal end portion of an endoscope. The distal end portion of the inner cylinder may project toward the distal end side of a distal end face of the endoscope. The slider may be configured to be movable along the axial direction by a fluid. The slider may be configured to be reciprocatable along the axial direction.

The slider may be composed of a plurality of slider pieces divided together with the annular groove.

The slider may be divided into the plurality of slider pieces along a plane including an axis of the slider or a plane perpendicular to the axis.

The distal end portion of the inner cylinder may include a tapered portion having an outer diameter decreasing toward the distal end.

A length of the tapered portion may be larger than a distance from a distal end of the slider to a proximal end of the annular groove.

A difference between an outer diameter at a proximal end of the tapered portion and an outer diameter at a distal end of the tapered portion may be larger than a difference between an outer diameter and an inner diameter of the ligation ring.

A part of the ligation ring may be temporarily fixed to a bottom face of the annular groove, and an inner peripheral side of the part may be in non-contact with an outer peripheral face of the inner cylinder.

A plurality of the annular grooves may be formed on the inner peripheral face of the slider, and a plurality of the ligation rings may be attached to the outer peripheral face of the inner cylinder such that the plurality of ligation rings correspond to the plurality of annular grooves.

The inner cylinder may be attachable around the endoscope distal end portion in an airtight or a liquidtight state.

The slider may be attached around the inner cylinder in an airtight or a liquidtight state.

A gap between the slider and the inner cylinder at a distal end portion and/or proximal end portion of the slider may be smaller than a thickness of the ligation ring.

A projection on which a distal end of the endoscope distal end portion is abuttable may be placed on the inner peripheral face of the inner cylinder. The inner cylinder and/or the slider may be made of a translucent material. An additional ligation ring located on the distal end side of the distal end of the slider and attached around the inner cylinder may be placed. In a state that the slider is disposed at a position opposite to the distal end portion side of the inner cylinder (state before moving the slider for ejecting the ligation ring), the ligation ring may be configured to be disposed at a position corresponding to the projection or a position opposite to the distal end portion side of the inner cylinder with respect to the projection. Incidentally, in a state that the slider is disposed at a position opposite to the distal end portion side of the inner cylinder (state before moving the slider for ejecting the ligation ring), the additional ligation ring may be configured to be disposed at a position corresponding to the projection or a position opposite to the distal end portion side of the inner cylinder with respect to the projection.

A slider extension portion projecting toward an outer peripheral side of the additional ligation ring may be placed on the distal end of the slider.

An inner gap may be formed between the inner cylinder and the slider, a first projection that projects into the inner gap is placed on the outer peripheral face of the inner cylinder, a second projection that projects into the inner gap is placed on an inner peripheral face of the slider, and a sum of heights of the first projection and the second projection in a radial direction of the slider may be structurally larger than a width of the inner gap in the radial direction.

A plurality of the first projections may be placed along the axial direction, and the radial-direction height of one of the plurality of first projections may be different from the radial-direction heights of the other remaining first projections. Alternatively, a plurality of the second projections may be placed along the axial direction, and the radial-direction height of one of the plurality of second projections may be different from the radial-direction heights of the other remaining second projections.

The plurality of first projections may be configured such that their radial-direction heights gradually increase toward the distal end side in the axial direction, or alternatively, the plurality of second projections may be configured such that their radial-direction heights gradually increase toward the proximal end side in the axial direction.

A first groove extending along the axial direction may be formed on the outer peripheral face of the inner cylinder, the first projections project from a bottom portion of the first groove toward the inner gap, and top portions of the second projections may be accommodated in the first groove, and/or a second groove extending along the axial direction may be formed on an inner peripheral face of the slider, the second projections may project from a bottom portion of the second groove toward the inner gap, and top portions of the first projections may be accommodated in the second groove.

An outer cylinder fixed to the inner cylinder and placed around the slider may be further provided.

An outer gap may be formed between the slider and the outer cylinder, a third projection that projects into the outer gap may be placed on an outer peripheral face of the slider, a fourth projection that projects into the outer gap is placed on an inner peripheral face of the outer cylinder, and a sum of heights of the third projection and the fourth projection in the radial direction of the slider may be structurally larger than a width of the outer gap in the radial direction.

A plurality of the third projections may be placed along the axial direction, and the radial-direction height of one of the plurality of third projections may be different from the radial-direction heights of the other remaining third projections. Alternatively, a plurality of the fourth projections may be placed along the axial direction, and the radial-direction height of one of the plurality of fourth projections may be different from the radial-direction heights of the other remaining fourth projections.

The plurality of third projections may be configured such that their radial-direction heights gradually increase toward the distal end side in the axial direction, or alternatively, the plurality of fourth projections may be configured such that their radial-direction heights gradually increase toward the distal end side in the axial direction.

A third groove extending along the axial direction may be formed on the outer peripheral face of the slider, the third projections project from a bottom portion of the third groove toward the outer gap, and top portions of the fourth projections may be accommodated in the thirteenth, and/or a fourth groove extending along the axial direction may be formed on the inner peripheral face of the outer cylinder, the fourth projections may project from a bottom portion of the fourth groove toward the outer gap, and top portions of the third projections may be accommodated in the fourth groove.

Fifth projections that project toward the inner peripheral face of the slider to abut on the inner peripheral face of the slider may be placed on the outer peripheral face of the inner cylinder, and/or sixth projections that project toward the outer peripheral face of the inner cylinder to abut on the outer peripheral face of the inner cylinder may be placed on the inner peripheral face of the slider.

The fifth projections and the sixth projections may extend along the axial direction.

Seventh projections that project toward the inner peripheral face of the outer cylinder to abut on the inner peripheral face of the outer cylinder may be placed on the outer peripheral face of the slider, and/or eighth projections that project toward the outer peripheral face of the slider to abut on the outer peripheral face of the slider may be placed on the inner peripheral face of the outer cylinder.

The seventh projections and the eighth projections may extend along the axial direction.

### ADVANTAGEOUS EFFECTS OF INVENTION

The present disclosure makes it possible to provide a ligation device that can always secure a good endoscopic visual field.

### BRIEF DESCRIPTION OF DRAWINGS

FIG. 1 (a) is a sectional view of a ligation device according to the first embodiment attached to an endoscope, illustrating a state that a slider is located on a proximal end side, and
FIG. 1 (b) is a sectional view of the ligation device according to the first embodiment attached to the endoscope, illustrating a state that the slider is advanced.
FIG. 2 (a) is a front view of one slider piece,
FIG. 2 (b) is a plan view of one slider piece,
FIG. 2 (c) is a side view of one slider piece,
FIG. 2 (d) is a front view of the other slider piece,
FIG. 2 (e) is a plan view of the other slider piece, and
FIG. 2 (f) is a side view of the other slider piece.
FIG. 3 (a) is a sectional view of a ligation device according to the second embodiment attached to an endoscope, illustrating a state that a slider is located on a proximal end side, and
FIG. 3 (b) is a sectional view of the ligation device according to the second embodiment attached to the endoscope, illustrating a state that the slider is advanced.
FIG. 4 is a partially cut-out sectional view of a ligation device including an endoscope portion according to the third embodiment.
FIG. 5 (a) is a front view of a slider according to a modification example, and
FIG. 5 (b) is a diagram illustrating a state that the slider according to the modification example is divided.
FIG. 6 is a diagram illustrating a state that a part of an outer periphery of a ligation ring is temporarily fixed to a bottom face of an annular groove using an adhesive.
FIG. 7 is an enlarged sectional view of a tapered portion of an inner cylinder according to a modification example.
FIG. 8 (a) is a sectional view of a ligation device according to the first modification example attached to an endoscope, illustrating a state that a slider is located on a proximal end side, and
FIG. 8 (b) is a sectional view of the ligation device according to the first modification example attached to the endoscope, illustrating a state that the slider is advanced.
FIG. 9 (a) is a sectional view of a ligation device according to the second modification example attached to an endoscope, illustrating a state that a slider is located on a proximal end side, and
FIG. 9 (b) is a sectional view of the ligation device according to the second modification example attached to the endoscope, illustrating a state that the slider is advanced.
FIG. 10 is a sectional view of a ligation device according to the third modification example attached to an endoscope, illustrating a state that a slider is located on a proximal end side.
FIG. 11 (a) is a partial sectional view of a ligation device according to the fourth modification example attached to an endoscope, illustrating a state that a slider is located on a proximal end side, and
FIG. 11 (b) is a partial sectional view taken along line XIb-XIb in FIG.11 (a).
FIG. 12 is a diagram explaining operations of the ligation device according to the fourth modification example.
FIG. 13 (a) is a partial sectional view of a ligation device according to the fifth modification example attached to an endoscope, illustrating a state that a slider is located on a proximal end side, and
FIG. 13 (b) is a partial sectional view taken along line XIIIb-XIIIb in FIG.13 (a).
FIG. 14 (a) is a partial sectional view of a ligation device according to the sixth modification example attached to an endoscope, illustrating a state that a slider is located on a proximal end side, and
FIG. 14 (b) is a partial sectional view taken along line XIVb-XIVb in FIG.14 (a).
FIG. 15 is a diagram illustrating a state that an inner cylinder according to the fifth modification example and the slider according to the sixth modification example are combined.
FIG. 16 (a) is a partial sectional view of the ligation device according to a modification example of the fourth modification example attached to the endoscope, illustrating a state that the slider is located on the proximal end side,
FIG. 16 (b) is a partial sectional view of the ligation device according to a modification example of the fifth modification example attached to the endoscope, illustrating a state that the slider is located on the proximal end side, and
FIG. 16 (c) is a partial sectional view of the ligation device according to a modification example of the sixth modification example attached to the endoscope, illustrating a state that the slider is located on the proximal end side.
FIG. 17 (a) is a partial sectional view of a ligation device according to the seventh modification example attached to an endoscope, illustrating a state that a slider is located on a proximal end side, and
FIG. 17 (b) is a partial sectional view taken along line XVIIb-XVIIb in FIG.17 (a).
FIG. 18 is a diagram explaining operations of the ligation device according to the seventh modification.
FIG. 19 (a) is a partial sectional view of a ligation device according to the eighth modification example attached to an endoscope, illustrating a state that a slider is located on a proximal end side, and
FIG. 19 (b) is a partial sectional view taken along line XIXb-XIXb in FIG.19 (a).
FIG. 20 (a) is a partial sectional view of a ligation device according to the ninth modification example attached to an endoscope, illustrating a state that a slider is located on a proximal end side, and
FIG. 20 (b) is a partial sectional view taken along line XXb-XXb in FIG.20 (a).
FIG. 21 is a diagram illustrating a state that the slider according to the eighth modification example and an outer cylinder according to the ninth modification example are combined.
FIG. 22 (a) is a partial sectional view of the ligation device according to a modification example of the seventh modification example attached to the endoscope, illustrating a state that the slider is located on the proximal end side,
FIG. 22 (b) is a partial sectional view of the ligation device according to a modification example of the eighth modification example attached to the endoscope, illustrating a state that the slider is located on the proximal end side, and
FIG. 22 (c) is a partial sectional view of the ligation device according to a modification example of the ninth modification example attached to the endoscope, illustrating a state that the slider is located on the proximal end side.
FIG. 23 (a) is a sectional view of a ligation device according to the tenth modification example attached to an endoscope, illustrating a state that a slider is located on a proximal end side, and
FIG. 23 (b) is a sectional view taken along line XXIII-XXIIIb in FIG.23 (a).
FIG. 24 (a) is a sectional view of a ligation device according to the eleventh modification example attached to an endoscope, illustrating a state that a slider is located on a proximal end side, and
FIG. 24 (b) is a sectional view taken along line XXIV-XXIVb in FIG.24 (a).

### DESCRIPTION OF EMBODIMENTS

Hereinafter, the disclosed embodiments will be explained with reference to the figures. Note that sizes of the ligation device illustrated in the figures are described to make it easier to understand the contents of the embodiments, and do not correspond to the actual sizes.

### <First Embodiment>

The disclosed first embodiment will be explained with reference to the figures.

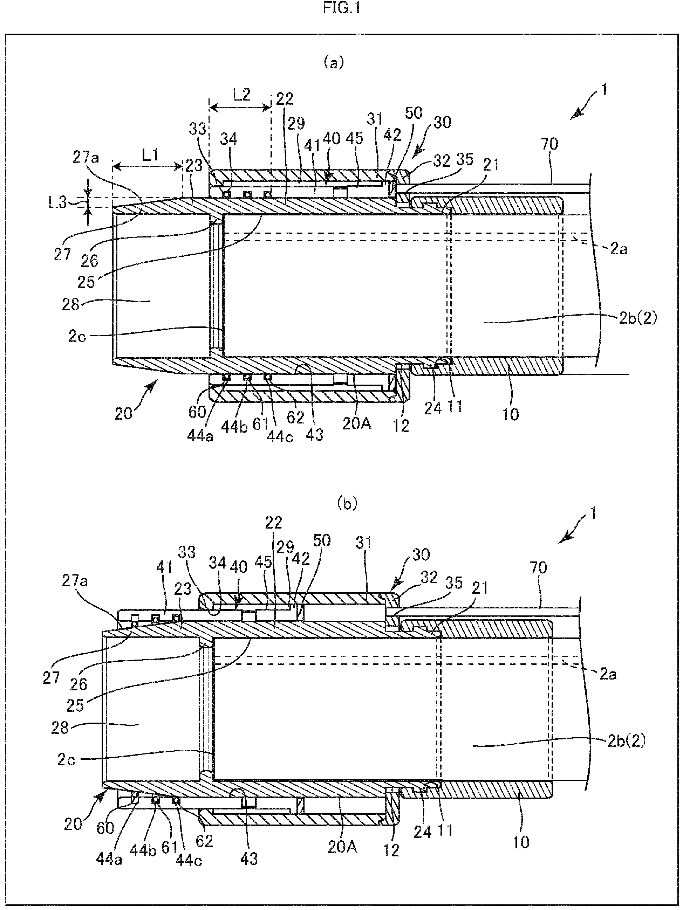

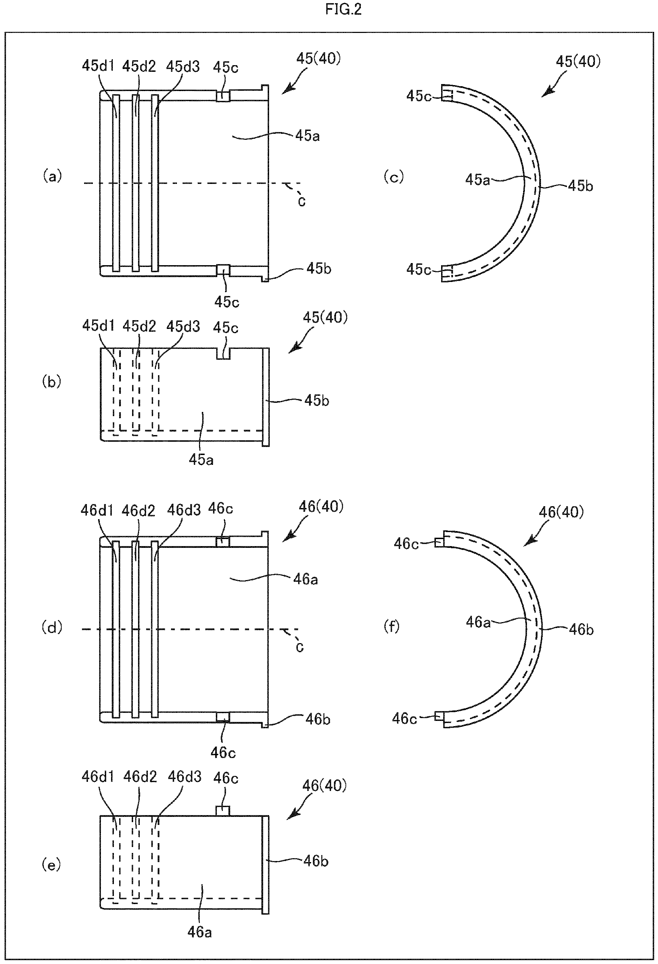

FIG. 1 (a) is a sectional view of a ligation device 1 according to the disclosed first embodiment attached to an endoscope 2, illustrating a state that a slider 40 is located on a proximal end side. FIG. 1 (b) is a sectional view of the ligation device 1 attached to the endoscope 2, illustrating a state that the slider 40 is advanced. FIG. 1 (a) and FIG. 1 (b) illustrate only a distal end portion of the endoscope 2 equipped with the ligation device 1. Note that, for the slider 40, only a slider piece 45 is illustrated (see FIG. 2), and for the endoscope 2, its appearance is illustrated, and a sectional view of the endoscope 2 is not illustrated (the same applies to the following figures).

Additionally, in FIG. 1 (a) and FIG. 1 (b), the left side of the figure is a distal end side (farther side) to be inserted into a body, and the right side is a proximal end side (hand side, nearer side). The endoscope 2 has a forceps hole 2a through which forceps not illustrated are inserted.

The ligation device 1 includes a joining member 10, an inner cylinder 20, an outer cylinder 30, the slider 40, a sealing member 50, ligation rings 60, 61 and 62, and a tube 70.

The joining member 10 has a hollow cylindrical shape and is placed for fixing the ligation device 1 to the endoscope 2, and pressure-joined to an outer periphery of the endoscope 2. An annular groove 11 is formed on a distal inner periphery of the joining member 10. A material constituting the joining member 10 is not particularly limited as long as the material has a strength for fixing the ligation device 1 to the endoscope 2 for preventing the ligation device from separating from the endoscope 2, and is biocompatible. For example, elastic materials such as a natural rubber, a synthetic rubber, and a thermoplastic elastomer can be used. Examples of the synthetic rubber include an isoprene rubber, a butadiene rubber, a styrene-butadiene rubber, a nitrile rubber, a butyl rubber, an ethylene-propylene rubber, an acrylic rubber, a fluorine rubber, a silicone rubber, and the like. In addition, examples of the thermoplastic elastomer include a styrene-based elastomer, an olefin-based elastomer, a polyester-based elastomer, a polyurethane-based elastomer, a polyamide-based elastomer, and the like.

The inner cylinder 20 is connected to a distal end portion of the joining member 10, and located on an endoscope distal end portion 2b, and attached to the endoscope distal end portion 2b in an airtight state or liquidtight state. The inner cylinder 20 includes a proximal end portion 21, an intermediate portion 22, and a distal end portion 23. The proximal end portion 21 is configured so as to be thinner than the intermediate portion 22 and have an outer diameter smaller than of the intermediate portion 22. On an outer periphery of the proximal end portion 21, an annular protruding portion 24 projecting outward is placed. The protruding portion 24 is fitted into the annular groove 11 of the joining member 10, so that the inner cylinder 20 is fixed to the joining member 10.

The intermediate portion 22 is located on the distal end side of the proximal end portion 21. On an inner peripheral face 25 of the intermediate portion 22, an annular projection 26 that projects inward is placed. A distal end of the endoscope distal end portion 2b abut on the projection 26 in an axial direction.

The distal end portion 23 is located on the distal end side of the intermediate portion 22. The distal end portion 23 projects toward the distal end side of a distal end face 2c of the endoscope 2. The distal end-side portion of the distal end portion 23 has a tapered portion 27 whose outer diameter gradually decreases toward the distal end. The distal end portion 23 and the distal end face 2c of the endoscope 2 constitute a recessed space 28.

A material constituting components such as the inner cylinder 20 is not particularly limited, and a metal material, a resin material, and a ceramic material can be used. Typical examples of the metal material include a stainless steel, titanium, and a nickel-titanium alloy. Examples of the resin material include a polyethylene, a polypropylene, a polyvinyl chloride, a polystyrene, an acrylic resin, a phenolic resin, a melamine resin, a polyimide, a polyamide, a polycarbonate, a polyether sulfone, a polyetheretherketone, and a polytetrafluoroethylene. Examples of the ceramic material include glass and fine ceramics.

Since the ligation rings 60, 61 and 62 are attached to the inner cylinder 20 as described later, typical examples of the material constituting the inner cylinder 20 are not particularly limited as long as the material has a strength enough to withstand the ligation rings 60, 61 and 62 and is biocompatible. The material constituting the inner cylinder 20 may be a translucent material, e.g. a polypropylene, a polycarbonate, a polyethersulfone, a polyimide, or an acrylic resin, for maintaining a wide visual field during treatment. Incidentally, the material constituting the inner cylinder 20 need not be a translucent material.

The outer cylinder 30 includes an outer cylindrical portion 31 placed around the intermediate portion 22 of the inner cylinder 20, and an outer cylinder cap 32. The inner cylinder 20 and the outer cylinder 30 constitute a slide space 29.

An annular protruding portion 33 projecting inward is placed on the distal end of the outer cylindrical portion 31. The protruding portion 33 and the intermediate portion 22 constitute an annular opening 34 where the slide space 29 is open. The outer cylinder cap 32 is bonded to the proximal end of the outer cylindrical portion 31 using an adhesive. An inner peripheral end of the outer cylinder cap 32 is fitted into a groove 12 composed of the proximal end of the intermediate portion 22 and the distal end of the joining member 10, so that movement of the outer cylinder 30 in the axial direction is restricted. The outer cylinder cap 32 closes the proximal end side of the slide space 29. A through-hole 35 communicating with the slide space 29 is formed on the outer cylinder cap 32. In the axial direction, the distal end of the outer cylindrical portion 31 is disposed at almost the same position as the projection 26 of the inner cylinder 20.

A material constituting the outer cylinder 30 is not particularly limited as long as the material has a strength against the pressure of the fluid and is biocompatible as described later, and for example, the materials cited as the material for the inner cylinder 20 can be used. In addition, a material for the adhesive is not particularly limited as long as the material has a strength against the pressure of the fluid and is biocompatible, and examples of the material include an acrylic resin-based adhesive, a urethane resin-based adhesive, an epoxy resin-based adhesive, a vinyl chloride resin solvent-based adhesive, a cyanoacrylate-based adhesive, a silicone-based adhesive, a phenol resin-based adhesive, and the like.

The slider 40 is cylindrical, placed between the inner cylinder 20 and the outer cylinder 30, and movably (e.g. reciprocably) attached around the inner cylinder 20 in an airtight or liquidtight state. On the distal end portion and/or proximal end portion of the slider 40, a gap between the slider 40 and the inner cylinder 20 is made smaller than thicknesses of the ligation rings 60, 61 and 62. An axial-direction length of the slider 40 is made substantially equal to the length of the outer cylindrical portion 31 of the outer cylinder 30.

The slider 40 includes a cylindrical portion 41 and a flange portion 42. On an inner peripheral face 43 of the cylindrical portion 41, three annular grooves 44a, 44b and 44c are formed at a predetermined interval along the axial direction. The annular grooves 44a, 44b and 44c are configured to have dimensions capable of accommodating the ligation rings 60, 61 and 62 described later. As illustrated in FIG. 1 (a) and FIG. 1 (b), when the slider 40 reciprocates with respect to the inner cylinder 20, the annular grooves 44a, 44b and 44c of the slider 40 advance and retract from the slide space 29 to the tapered portion 27. In a state that the slider 40 is located on the most distal end side, the flange portion 42 abuts on the protruding portion 33, and the slider 40 is prevented from coming out from the slide space 29. Incidentally, the slider 40 may be configured only to be able to advance with respect to the inner cylinder 20.

In addition, as illustrated in FIG. 2 (a) to FIG. 2 (f), the slider 40 is composed of a plurality of (two in the first embodiment) slider pieces 45 and 46 divided together with the annular grooves 44a, 44b and 44c along a plane including an axis c of the slider 40. In the first embodiment, the slider 40 is composed of two slider pieces 45 and 46 obtained by half-cutting a hollow cylinder along the plane including the axis of the slider 40. Each of the slider pieces 45 and 46 includes half-cylindrical portions 45a and 46a corresponding to the cylindrical portion 41, and half-flange portions 45b and 46b corresponding to the flange portion 42, respectively. Each of the half-cylindrical portions 45a and 46a has half-annular grooves 45d1, 45d2, 45d3, 46d1, 46d2 and 46d3 corresponding to the annular grooves 44a, 44b and 44c. In addition, a pair of fitting recessed portions 45c are formed on a half-cut face of the half-cylindrical portion 45a, and a pair of fitting protruding portions 46c are formed on the half-cylindrical portion 46a. The pair of fitting protruding portions 46c are fitted into the pair of fitting recessed portions 45c, and they are bonded to each other using an adhesive, so that the slider pieces 45 and 46 are integrated to form the slider 40.

A material constituting the slider 40 is not particularly limited as long as the material has excellent slidability to the inner cylinder 20 and is biocompatible, and the materials cited as the materials for the inner cylinder 20 can be used. The material constituting the slider 40 may be a translucent material, e.g. a polypropylene, a polycarbonate, a polyethersulfone, a polyimide, an acrylic resin, or the like for maintaining a wide visual field during treatment. Incidentally, the material constituting the slider 40 need not be a translucent material. In addition, a material for the adhesive is not particularly limited as long as the material has a strength against the pressure of the fluid and is biocompatible, and examples of the material include an acrylic resin-based adhesive, a urethane resin-based adhesive, an epoxy resin-based adhesive, a vinyl chloride resin solvent-based adhesive, a cyanoacrylate-based adhesive, a silicone-based adhesive, a phenol resin-based adhesive, and the like.

The sealing member 50 has an annular shape and is placed so as to be slidable in the slide space 29 while abutting on the inner periphery of the outer cylinder cap 32 and the outer periphery of the inner cylinder 20. This sealing member 50 is fixed to the proximal end of the slider 40 so as to be movable together with the slider 40. Thereby a space surrounded by the sealing member 50, the outer cylinder cap 32, the inner cylinder 20, and the outer cylindrical portion 31 is airtightly preserved. The material constituting the sealing member 50 is not particularly limited as long as the material can preserve the space in the airtight state and is biocompatible. For example, elastic materials such as a natural rubber, a synthetic rubber, and a thermoplastic elastomer can be used. Examples of the synthetic rubber include an isoprene rubber, a butadiene rubber, a styrene-butadiene rubber, a nitrile rubber, a butyl rubber, an ethylene-propylene rubber, an acrylic rubber, a fluorine rubber, a silicone rubber, and the like. In addition, examples of the thermoplastic elastomer include a styrene-based elastomer, an olefin-based elastomer, a polyester-based elastomer, a polyurethane-based elastomer, a polyamide-based elastomer, and the like.

The ligation rings 60, 61 and 62 are O-rings, which are attached to the outer periphery of the inner cylinder 20 and located inside the annular grooves 44a, 44b and 44c. When the slider 40 is disposed at a position opposite to the side of the distal end portion 23 of the inner cylinder 20 (most proximal end side), the ligation rings 60, 61 and 62 are disposed at a position opposite to the side of the distal end portion 24 of the inner cylinder 20 with respect to the projection 26. A material constituting the ligation rings 60, 61 and 62 is not particularly limited as long as the material sufficiently extends so as to be attachable to the inner cylinder 20 and has a ligation force sufficient to necrotize an affected area, and is biocompatible. For example, elastic materials such as a natural rubber, a synthetic rubber, and a thermoplastic elastomer can be used. Examples of the synthetic rubber include an isoprene rubber, a butadiene rubber, a styrene-butadiene rubber, a nitrile rubber, a butyl rubber, an ethylene-propylene rubber, an acrylic rubber, a fluorine rubber, a silicone rubber, and the like. In addition, examples of the thermoplastic elastomer include a styrene-based elastomer, an olefin-based elastomer, a polyester-based elastomer, a polyurethane-based elastomer, a polyamide-based elastomer, and the like. Incidentally, the materials constituting the ligation rings 60, 61 and 62 may include e.g. a radiopaque material such as gold, platinum, tungsten, an alloy containing these elements (e.g. a platinum-nickel alloy, or the like), barium sulfate, bismuth subcarbonate, bismuth trioxide, bismuth oxychloride, and bismuth subcarbonate, or a powder of the radiopaque material. Sectional shapes of the ligation rings 60, 61 and 62 are not limited to circle, and may be another shape such as rectangle. A color of the ligation rings 60, 61 and 62 is preferably a color such as black, which is distinct from a surrounding tissue.

The tube 70 extends from the distal end portion to the proximal end portion along the endoscope 2. A syringe, not illustrated, for delivering the fluid such as air to the slider 40 is connected to the proximal end of the tube 70. The distal end of the tube 70 is airtightly connected to the proximal end of the through-hole 35 of the outer cylinder cap 32. The material constituting the tube 70 is not particularly limited as long as the material has flexibility capable of following deformation of the endoscope 2 and is biocompatible, and examples of the material include a polyethylene, a polypropylene, a polyvinyl chloride, a fluororesin, and the like.

As illustrated in FIG. 1 (a), the tapered portion 27 is configured to have a length L1 larger than a distance L2 from the distal end of the slider 40 to a proximal end of the annular groove 11 on the most proximal end side. In addition, the tapered portion 27 is configured such that a difference L3 between a proximal outer diameter and a distal outer diameter of a slope 27a is larger than a difference between the outer diameter and the inner diameter of the ligation rings 60, 61 and 62. Herein, the outer diameters and inner diameters of the ligation rings 60, 61 and 62 mean the outer diameters and inner diameters in a state that the ligation rings 60, 61 and 62 are attached around the inner cylinder 20.

A whole length of the ligation device 1 (length from the distal end of the inner cylinder 20 to the proximal end of the joining member 10) is set to e.g. 20 to 25 mm. In an inner cylinder 20b (part without the projection 26), for example, an inner diameter is set to 8 to 16 mm, and an outer diameter (maximum diameter) is set to 11 to 25 mm.

Next, an example of how to use the ligation device 1 will be explained. Herein, a method of ligating a diverticula formed on a wall face of a digestive tract will be explained as an example.

First, as illustrated in FIG. 1 (a), in a state that the whole slider 40 is located on the slide space 29, the endoscope 2 equipped with the ligation device 1 is inserted into the digestive tract, and an inside of the digestive tract is observed. A bleeding diverticula is identified, the endoscope 2 and the ligation device 1 are made close to the diverticula, the diverticula is sucked through the forceps hole 2a, the diverticula is reversed so as to protrude toward the endoscope 2, and the diverticula is located in the recessed space 28. As illustrated in FIG. 1 (b), a fluid is delivered to the slide space 29 through the tube 70 by operating the syringe not illustrated, and the slider 40 and the sealing member 50 are advanced using a pressure of the fluid as a driving source. The slider 40 moves to the distal end side of the inner cylinder 20, and the ligation ring 60 located on the most distal end moves to the tapered portion 27. Thereby, the ligation ring 60 moves to the distal end side on the tapered portion 27 while the diameter of the ligation ring 60 gradually decreases, and the ligation ring 60 is ejected from the tapered portion 27, and the sucked diverticula is ligated by the ligation ring 60. Then, the endoscope 2 equipped with the ligation device 1 is taken out of the digestive tract. After that, the ligated diverticula necrotizes and is discharged to the outside of the body together with the ligation ring 60.

The ligation device 1 according to the first embodiment is configured such that the ligation rings 60, 61 and 62 are located in the annular grooves 44a, 44b and 44c of the slider 40, and the slider 40 can reciprocate along the axial direction of the inner cylinder 20. Thereby, if the slider 40 and the ligation rings 60, 61 and 62 are advanced in an attempt to ligate an affected area but the ligation is aborted, the slider 40 can be retracted together with the ligation rings 60, 61 and 62. Thus, when moving the endoscope 2 to another affected area, the slider 40 and the ligation rings 60, 61 and 62 can be removed from a visual field of the endoscope 2, so that a good visual field can be secured. In addition, when ligating another affected area, an advancement amount of the slider can be accurately adjusted (the slider can be accurately advanced to a predetermined position) by retracting the slider 40 to a predetermined position, e.g. a position illustrated in FIG. 1 (a). In addition, when ligating another affected area, new ligation can be performed by advancing the slider 40.

Since the slider 40 is composed of the plurality of slider pieces 45 and 46 divided together with the annular grooves 44a, 44b and 44c, the slider 40 can be formed around the inner cylinder 20 such that the ligation rings 60, 61 and 62 can be fitted within the annular grooves 44a, 44b and 44c on the inner cylinder 20 equipped with the ligation rings 60, 61 and 62.

Since the slider 40 is divided into the plurality of slider pieces 45 and 46 along a plane including the axis of the slider 40, the slider 40 can be easily placed around the inner cylinder 20 such that the ligation rings 60, 61 and 62 can be fitted within the annular grooves 44a, 44b and 44c on the inner cylinder 20 equipped with the ligation rings 60, 61 and 62. In addition, the slider 40 can be easily prepared.

The distal end portion 23 of the inner cylinder 20 includes the tapered portion 27 whose outer diameter gradually decreases toward the distal end, and therefore, when the slider 40 moves to the distal end side of the inner cylinder 20 and the ligation rings 60, 61 and 62 move to the tapered portion 27, the ligation rings 60, 61 and 62 move to the distal end side of the distal end portion 23 on the tapered portion 27 while diameters of the ligation rings 60, 61 and 62 gradually decrease, and the ligation rings are ejected from the distal end portion 23, so that the affected area can be ligated.

Since the tapered portion is configured to have the length L1 larger than the distance L2 from the distal end of the slider 40 to the proximal end of the annular groove 11 on the most proximal end side, all of the ligation rings 60, 61 and 62 can move to the distal end side of the inner cylinder 20 along the tapered portion 27 while the diameters of the ligation rings gradually decrease before the distal end of the slider 40 projects toward the distal end side with respect to the distal end of the inner cylinder 20, so that the affected area can be securely ligated.

Since the tapered portion 27 is configured such that the difference L3 between the proximal outer diameter and the distal outer diameter of the slope 27a is larger than the difference between the outer diameter and the inner diameter of the ligation rings 60, 61 and 62, the ligation rings 60, 61 and 62 can be securely ejected on the tapered portion 27.

The plurality of annular grooves 44a, 44b and 44c are formed on the inner peripheral face 43 of the slider 40, and the plurality of ligation rings 60, 61 and 62 are attached to an outer peripheral face 20A of the inner cylinder 20 such that the ligation rings 60, 61 and 62 correspond to the plurality of annular grooves 44a, 44b and 44c respectively. Thus, ligation can be continuously performed without taking the ligation device 1 out of the body. In addition, when ligation of the first affected area is completed and the endoscope 2 is moved to another affected area, the slider 40 and the ligation rings 61 and 62 can be removed from the visual field of the endoscope 2 by retracting the slider 40 together with the ligation rings 61 and 62, so that a good visual field can be secured. In addition, when ligating another affected area, the slider 40 is advanced again, so that ligation can be performed by the ligation rings 61 and 62.

Since the inner cylinder 20 is attached to the endoscope distal end portion 2b in an airtight or liquidtight state, a force enough to suck the affected area into the recessed space 28 composed of the distal end portion 23 of the inner cylinder 20 and the endoscope distal end portion 2b can be generated.

On the distal end portion and/or the proximal end portion of the slider 40, the gap between the slider 40 and the inner cylinder 20 is configured to be smaller than the thicknesses of the ligation rings 60, 61 and 62, and therefore the ligation rings 60, 61 and 62 can be securely moved together with the slider 40, so that the affected area can be securely ligated.

Since the inner peripheral face 25 of the inner cylinder 20 has the projection 26 on which the distal end of the endoscope distal end portion 2b abuts, the projection 26 makes it easier to position the ligation device 1 with respect to the endoscope distal end portion 2b.

The inner cylinder 20 and/or the slider 40 is made of a translucent material, so that a wide visual field during treatment using the endoscope 2 can be maintained.

### <Second Embodiment>

The disclosed second embodiment will be explained with reference to the figures.

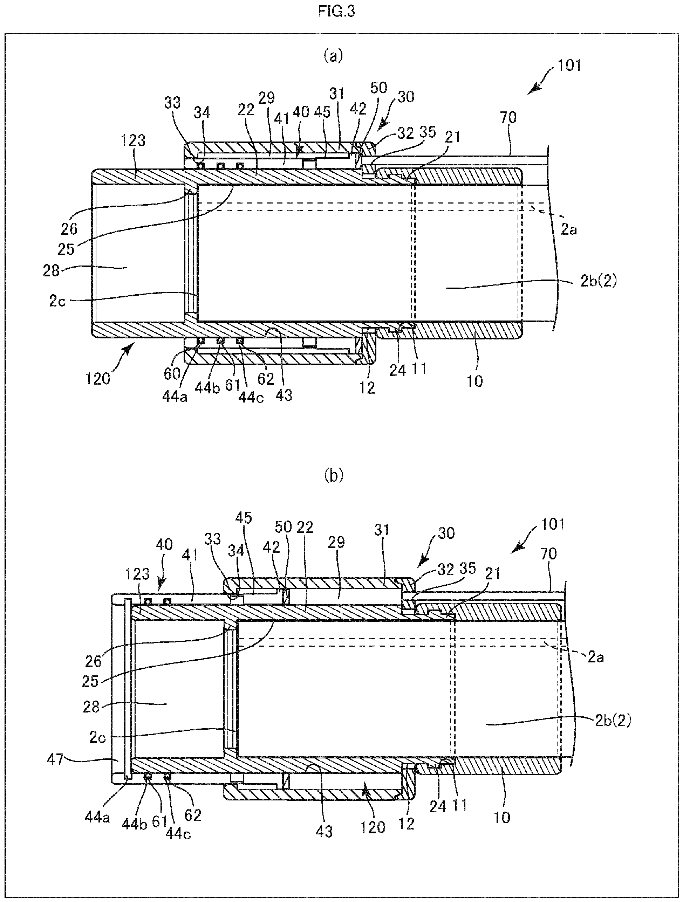

FIG. 3 (a) is a sectional view of a ligation device 101 according to the disclosed second embodiment attached to the endoscope 2, illustrating a state that the slider 40 is located on the proximal end side. FIG. 3 (b) is a sectional view of the ligation device 101 attached to the endoscope 2, illustrating a state that the slider 40 is advanced. FIG. 3 (a) and FIG. 3 (b) illustrate only the distal end portion of the endoscope 2 equipped with the ligation device 101.

Additionally, in FIG. 3 (a) and FIG. 3 (b), the left side of the figure is a distal end side (farther side) to be inserted into a body, and the right side is a proximal end side (hand side, nearer side). The endoscope 2 has a forceps hole 2a through which forceps not illustrated are inserted.

Note that, in the ligation device 101 according to the second embodiment, the same members as in the ligation device 1 according to the first embodiment are given the same reference numerals as in the first embodiment, and detailed explanation of the same members is omitted.

The ligation device 101 includes the joining member 10, an inner cylinder 120, the outer cylinder 30, the slider 40, the sealing member 50, the ligation rings 60, 61 and 62, and the tube 70.

As illustrated in FIG. 3 (a) and FIG. 3 (b), in the inner cylinder 120 according to the second embodiment, the distal end portion 123 has no tapered portion. That means, the distal end portion 123 is configured to have a thickness that is substantially constant from the proximal end to the distal end. In this configuration, as illustrated in FIG. 3 (b), when the ligation rings 60, 61 and 62 are ejected from the distal end portion 123 of the inner cylinder 120, the annular grooves 44a, 44b and 44c are located closer to the distal end side than the distal end of the distal end portion 123 of the inner cylinder 120. That means, the distal end portion of the slider 40 projects closer to the distal end side than the distal end of the distal end portion 123 of the inner cylinder 120. Thereby, the slider 40, the inner cylinder 120, and the endoscope distal end portion 2b constitute a large recessed space 47.

In addition, since the slider 40 is attached around the inner cylinder 120 in an airtight or liquidtight state, a force enough to suck an affected area into the recessed space 47 composed of the slider 40, the inner cylinder 120 and the endoscope distal end portion 2b can be generated even in a state that the slider 40 is located on the distal end side of the inner cylinder 120. Furthermore, a larger volume of affected area can be contained in the recessed space 47.

### <Third Embodiment>

The disclosed third embodiment will be explained with reference to the figures.

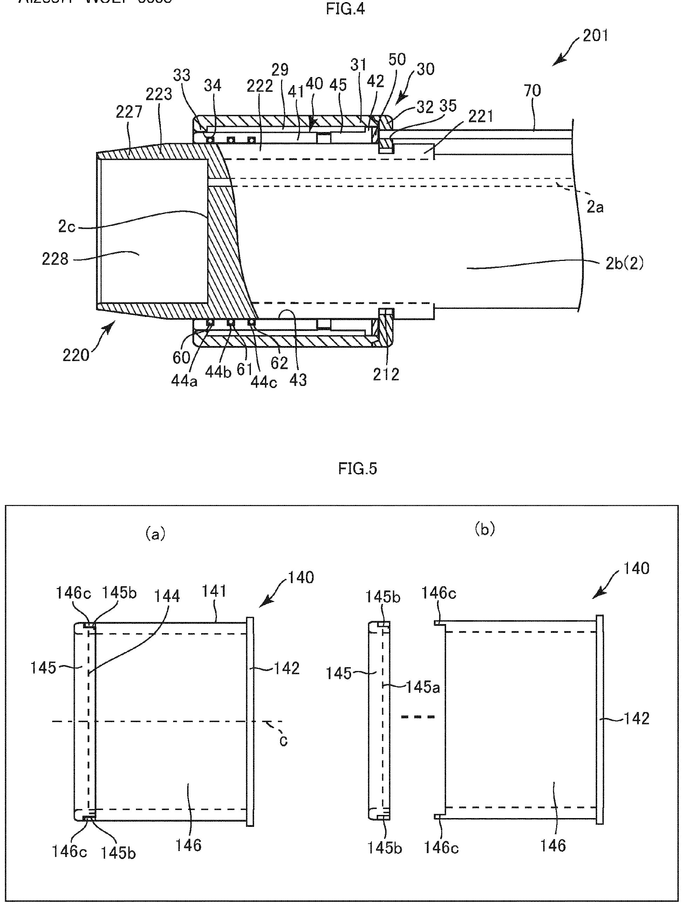

FIG. 4 is a partially cut-out sectional view of a ligation device 201 according to the third embodiment, including the endoscope portion 2. FIG. 4 illustrates only a distal end portion of the ligation device 201 including an endoscope portion 2.

Additionally, in FIG. 4, the left side of the figure is a distal end side (farther side) to be inserted into a body, and the right side is a proximal end side (hand side, nearer side).

Note that, in the ligation device 201 according to the third embodiment, the same members as in the ligation device 1 according to the first embodiment are given the same reference numerals as in the first embodiment, and detailed explanation of the same members is omitted.

The ligation device 201 includes an inner cylinder 220, the endoscope portion 2, the outer cylinder 30, the slider 40, the sealing member 50, the ligation rings 60, 61 and 62, and the tube 70.

The inner cylinder 220 is located on the endoscope distal end portion 2b and is formed integrally with the endoscope distal end portion 2b. The inner cylinder 220 includes a proximal end portion 221, an intermediate portion 222, and a distal end portion 223. The intermediate portion 222 is located on the distal end side of the proximal end portion 221. The inner peripheral end of the outer cylinder cap 32 is fitted into a groove 212 between the intermediate portion 222 and the proximal end portion 221.

The distal end portion 223 is located on the distal end side of the intermediate portion 222. The distal end portion 223 projects toward the distal end side of the distal end face 2c of the endoscope portion 2. The distal end-side portion of the distal end portion 223 has a tapered portion 227 whose outer diameter gradually decreases toward the distal end. The distal end portion 223 and the distal end face 2c of the endoscope 2 constitute a recessed space 228. A configuration such as a length of the tapered portion 227 is the same as of the tapered portion 27 according to the first embodiment.

The ligation device 201 having such a configuration can also exhibit the same effect as of the ligation device 1 according to the first embodiment.

Although the disclosed embodiments have been described above, the present disclosure is not limited to the aforementioned embodiments and can be variously modified.

For example, in the aforementioned embodiments, although the slider 40 is composed of two slider pieces 45 and 46 obtained by cutting a hollow cylinder into half along the plane including the axis c of the slider 40, a slider 140 may be divided into a plurality of (two in this modification example) hollow cylindrical slider pieces 145 and 146 along a plane perpendicular to the axis c of the slider 140, as illustrated in FIG. 5 (a) and FIG. 5 (b). Preferably, the position for the division faces the annular groove.

The slider piece 145 has an annular notch 145a and a pair of fitting recessed portions 145b on the right side in FIG. 5 (b). The slider piece 146 has a pair of fitting protruding portions 146c on the left side in FIG. 5 (b). The pair of fitting protruding portions 146c are fitted into the pair of fitting recessed portions 145b, and they are bonded to each other using an adhesive, so that the slider pieces 145 and 146 are integrated, the annular groove 144 is formed, and the slider 140 having a cylindrical portion 141 and a flange 142 is formed. Incidentally, although the sliders 40 and 140 are divided into two pieces in the aforementioned embodiments and modification examples, the sliders 40 and 140 may be divided into three or more pieces.

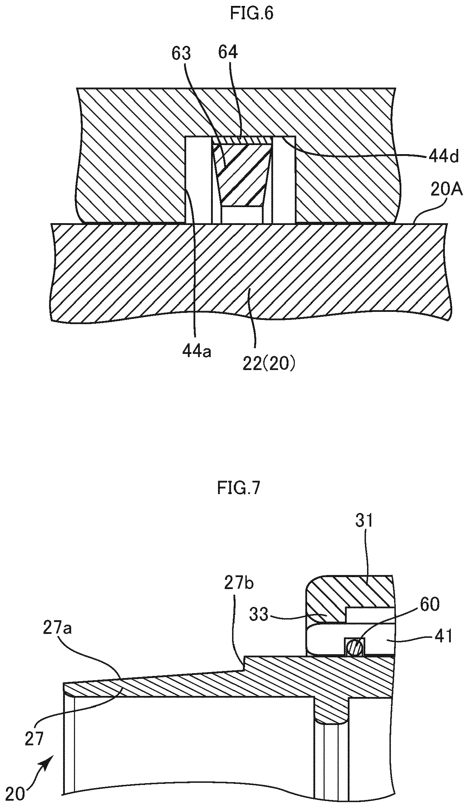

In addition, as illustrated in FIG. 6, a ligation ring 63 having a rectangular sectional shape is adopted instead of the ligation rings 60, 61 and 62, and a part of an outer periphery of the ligation ring 63 may be temporarily fixed to a bottom face (inner peripheral face) 44d constituting the annular groove 44a using an adhesive 64. Herein, an inner periphery of the ligation ring 63 may or may not come into contact with the outer peripheral face 20A of the intermediate portion 22 of the inner cylinder 20.

According to this configuration, a frictional resistance of the ligation ring 63 to the inner cylinder 20 decreases, and therefore the slider 40 can be smoothly moved. A material constituting the adhesive 64 is not particularly limited as long as the material has an adhesivity by which a part of the outer periphery of the ligation ring 63 is bonded to the bottom face (inner peripheral face) 44d of the annular groove 44a in a state that the ligation ring 63 is attached to the intermediate portion 22 of the inner cylinder 20, and the part of the outer periphery of the ligation ring 63 leaves the bottom face (inner peripheral face) 44d of the annular groove 44a by a diameter-reducing force of the ligation ring 63 when the ligation ring 63 moves to the tapered portion 27, and the material is biocompatible. Examples of the material include an acrylic resin-based adhesive, a urethane resin-based adhesive, an epoxy resin-based adhesive, a vinyl chloride resin solvent-based adhesive, a cyanoacrylate-based adhesive, a silicone-based adhesive, a phenolic resin-based adhesive, and the like.

In addition, although three ligation rings 60, 61 and 62 are placed in the aforementioned embodiments, it is only necessary to place one or more ligation rings. In addition, although the fluid for driving the slider 40 is air, the fluid may be a liquid such as physiological saline. Incidentally, as the ligation ring, for example, a ring-shaped member made of a shape-memory alloy can be used. When such a ring-shaped member is ejected from the annular groove of the slider, the shape of the ring-shaped member changes from circle to non-circle, and therefore ligation in accordance with a shape of the affected area is possible. Also, a clip made of a shape-memory alloy can be used as the ligation ring. When accommodated in the annular groove of the slider, such a clip is ring-shaped, and when ejected from the annular groove of the slider, the clip is transformed into a previously-memorized shape.

In addition, the aforementioned embodiments have a configuration that the outer cylinder 30 is placed around the inner cylinder 20 to form a slide space 29, and the slider 40 is driven by a fluid, but a configuration that a hydraulic cylinder is placed instead of the outer cylinder 30 and the sealing member 50, and the slider 40 is driven by the hydraulic cylinder may be adopted.

In addition, as illustrated in FIG. 7, a level difference portion 27b may be formed on the proximal end of the tapered portion 27. Preferably, a radial-direction width of the level difference portion 27b is larger than the difference between the inner diameter and the outer diameter of the ligation rings 60, 61 and 62. According to this configuration, when the ligation rings 60, 61 and 62 move to the level difference portion 27b, the ligation rings 60, 61 and 62 decrease in diameter and leave the slider 40, then the ligation rings 60, 61 and 62 move to the distal end side of the distal end portion 23 on the tapered portion 27 while the diameters of the ligation rings gradually decrease, and are ejected from the distal end portion 23, so that the affected area can be ligated. If the level difference portion 27b is formed in such a way, it becomes easy to form a tapered portion having the length L1 larger than the distance L2 from the distal end of the slider 40 to the proximal end of the annular groove 11 on the most proximal end side, and the ligation rings 60, 61 and 62 can be ejected from the distal end portion 23 while the distal end of the slider 40 does not project toward the distal end side from the distal end of the inner cylinder 20, so that the affected area can be securely ligated.

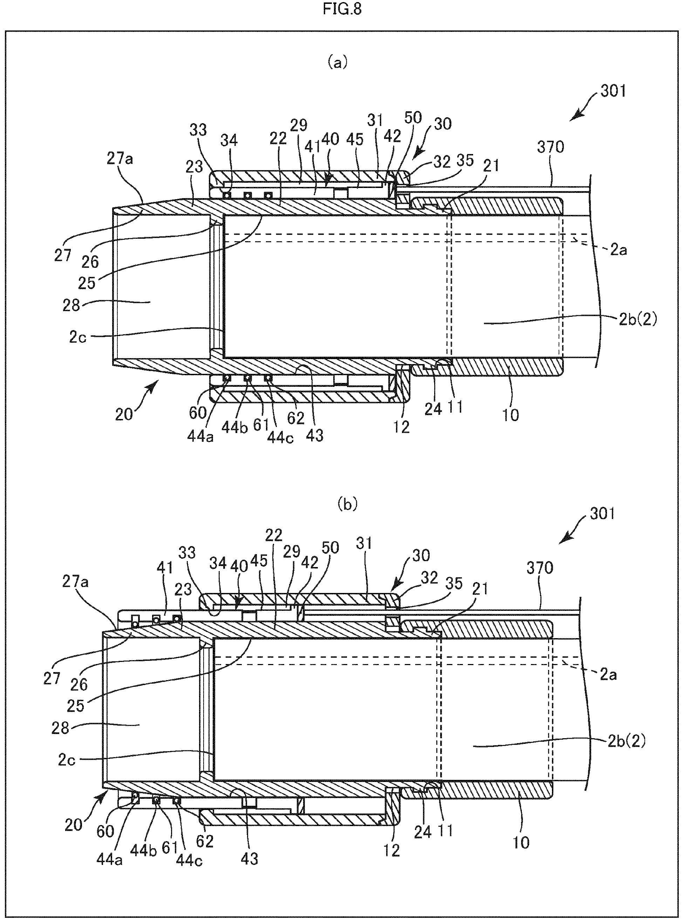

FIG. 8 (a) is a sectional view of a ligation device 301 according to the first modification example attached to the endoscope 2, illustrating a state that the slider 40 is located on the proximal end side. FIG. 8 (b) is a sectional view of the ligation device 301 attached to the endoscope 2, illustrating a state that the slider 40 is advanced. FIG. 8 (a) and FIG. 8 (b) illustrate only the distal end portion of the endoscope 2 equipped with the ligation device 301.

Additionally, in FIG. 8 (a) and FIG. 8 (b), the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 301 according to the first modification example, the same members as in the ligation device 1 according to the first embodiment are given the same reference numerals as in the first embodiment, and detailed explanation of the same members is omitted.

The ligation device 301 includes the joining member 10, the inner cylinder 20, the outer cylinder 30, the slider 40, the sealing member 50, the ligation rings 60, 61 and 62, and a rod 370.

The rod 370 extends from the distal end portion to the proximal end portion along the endoscope 2. The distal end of the rod 370 is inserted through the through-hole 35 of the outer cylinder cap 32 and connected to the sealing member 50. The proximal end portion of the rod 370 can be operated by an operator. When the operator pushes and pulls the proximal end portion of the rod 370, the slider 40 advances and retracts together with the sealing member 50.

In the first modification example, when the rod 370 is pushed, the slider 40 advances from the state illustrated in FIG. 8 (a) and moves to the distal end side of the inner cylinder 20 as illustrated in FIG. 8 (b). As a result, the ligation ring 60 located on the most distal end moves to the tapered portion 27, the ligation ring 60 located on the most distal end moves to the tapered portion 27, the ligation ring 60 is ejected from the tapered portion 27, and a sucked diverticula is ligated by the ligation ring 60. Then, the rod 370 is pulled, so that the advanced slider 40 and the ligation rings 61 and 62 can be retracted to a predetermined position e.g. a position illustrated in FIG. 8 (a).

The ligation device 301 having such a configuration can also exhibit the same effect as of the ligation device 1 according to the first embodiment.

Preferably, the material constituting the rod 370 has sufficient tensile strength and rigidity from the viewpoint of preventing the rod 370 itself from being cut and securely advancing and retracting the slider 40. Examples of the material include a metal material such as a stainless steel like SUS304, a nickel-titanium alloy, and a cobalt-chromium alloy, and the like. Additionally, in the first modification example, the sealing member 50 need not be provided, and the rod 370 may be directly connected to the proximal end of the slider 40.

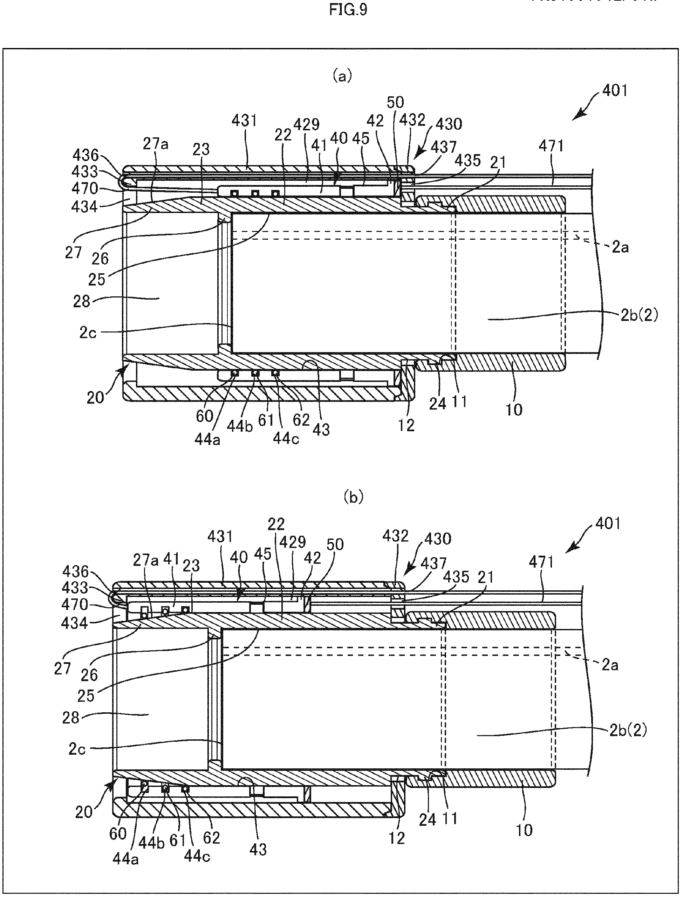

FIG. 9 (a) is a sectional view of a ligation device 401 according to the second modification example attached to the endoscope 2, illustrating a state that the slider 40 is located on the proximal end side. FIG. 9 (b) is a sectional view of the ligation device 401 attached to the endoscope 2, illustrating a state that the slider 40 is advanced. FIG. 9 (a) and FIG. 9 (b) illustrate only the distal end portion of the endoscope 2 equipped with the ligation device 401.

Additionally, in FIG. 9 (a) and FIG. 9 (b), the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 401 according to the second modification example, the same members as in the ligation device 1 according to the first embodiment are given the same reference numerals as in the first embodiment, and detailed explanation of the same members is omitted.

The ligation device 401 includes the joining member 10, the inner cylinder 20, an outer cylinder 430, the slider 40, the sealing member 50, the ligation rings 60, 61 and 62, a first wire 470, and a second wire 471.

The outer cylinder 430 includes an outer cylindrical portion 431 placed around the intermediate portion 22 and the distal end portion 23 of the inner cylinder 20, and an outer cylinder cap 432. The inner cylinder 20 and the outer cylinder 430 constitute a slide space 429.

The distal end of the outer cylindrical portion 431 is disposed at almost the same position as the distal end of the inner cylinder 20 in the axial direction. On the distal end of the outer cylindrical portion 431, an annular protruding portion 433 that projects inward is placed. The protruding portion 433 and the distal end portion 23 constitute an annular opening 434 through which a slide space 429 opens. The outer cylindrical portion 431 has a first through-hole 436 that penetrates from the distal end to the proximal end.

The outer cylinder cap 432 is bonded to the proximal end of the outer cylindrical portion 431 using an adhesive. An inner peripheral end of the outer cylinder cap 32 is fitted into the groove 12 composed of the proximal end of the intermediate portion 22 and the distal end of the joining member 10, so that movement of the outer cylinder 430 in the axial direction is restricted. The outer cylinder cap 432 closes the proximal end side of the slide space 429. The outer cylinder cap 432 has a second through-hole 435 and a third through-hole 437. The third through-hole 437 is formed outside the second through-hole 435 and communicates with the proximal end of the first through-hole 436. The second through-hole 435 communicates with the slide space 429.

The first wire 470 extends from the distal end portion to the proximal end portion along the endoscope 2. One end of the first wire 470 is connected to the distal end of the cylindrical portion 41 of the slider 40. The first wire 470 extends from the portion connected to the cylindrical portion 41 toward the distal end side, passes through the opening 434, turns back along the distal end of the outer cylindrical portion 431, passes through the first through-hole 436 of the outer cylindrical portion 431 and the third through-hole 437 of the outer cylinder cap 432, and extends to the proximal end portion of the endoscope 2. The proximal end of the first wire 470 can be operated by an operator. When the wire 470 is pulled by the operator, the slider 40 is pulled and advanced.

The second wire 471 extends from the distal end portion to the proximal end portion along the endoscope 2. The distal end of the second wire 471 is inserted through the second through-hole 435 of the outer cylinder cap 432 and connected to the sealing member 50. The proximal end of the second wire 471 is configured to be operable by an operator. When the proximal end of the second wire 471 is pulled by the operator, the slider 40 is pulled together with the sealing member 50 and retracts.

In the second modification example, when the first wire 470 is pulled, the slider 40 is pulled and advances from the state illustrated in FIG. 9 (a), and moves to the distal end side of the inner cylinder 20 as illustrated in FIG. 9 (b). As a result, the ligation ring 60 located on the most distal end moves to the tapered portion 27, the ligation ring 60 located on the most distal end moves to the tapered portion 27, the ligation ring 60 is ejected from the tapered portion 27, and a sucked diverticula is ligated by the ligation ring 60. Then, the second wire 471 is pulled, so that the advanced slider 40 and the ligation rings 61 and 62 can be retracted to a predetermined position e.g. a position illustrated in FIG. 9 (a).

The ligation device 401 having such a configuration can also exhibit the same effect as of the ligation device 1 according to the first embodiment.

As the first wire 470 and the second wire 471, for example, a known wire or rope made of a metal material or a resin material can be used. Additionally, in the second modification example, the sealing member 50 need not be provided, and the second wire 471 may be connected directly to the proximal end of the slider 40.

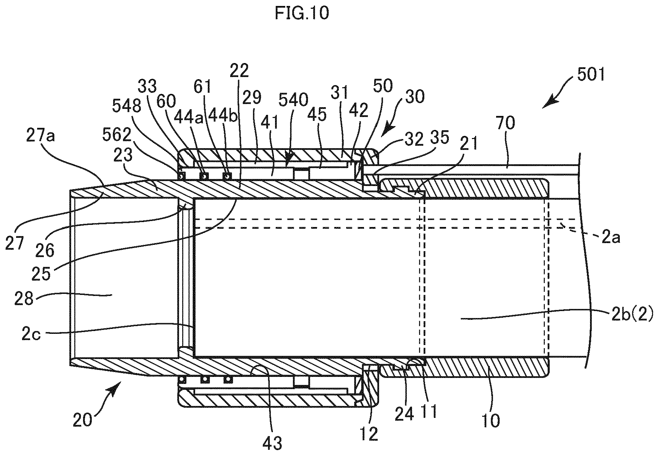

FIG. 10 is a sectional view of a ligation device 501 according to the third modification example attached to the endoscope 2, illustrating a state that a slider 540 is located on a proximal end side. FIG. 10 illustrates only the distal end portion of the endoscope 2 equipped with the ligation device 501.

Additionally, in FIG. 10, the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 501 according to the third modification example, the same members as in the ligation device 1 according to the first embodiment are given the same reference numerals as in the first embodiment, and detailed explanation of the same members is omitted.

The ligation device 501 includes the joining member 10, the inner cylinder 20, the outer cylinder 30, the slider 540, the sealing member 50, ligation rings 60, 61 and 562, and the tube 70.

In the third modification example, two annular grooves 44a and 44b are formed on the inner peripheral face 43 of the slider 40, and the ligation rings 60 and 61 are attached to the outer peripheral face 20A of the inner cylinder 20 such that the ligation rings 60 and 61 correspond to the annular grooves 44a and 44b respectively. In addition, as an additional ligation ring, the ligation ring 562 located on the distal end side of the distal end of the slider 540 and attached around the inner cylinder 20 is placed. On the distal end of the slider 540, a slider extension portion 548 that projects toward the outer peripheral side of the ligation ring 562 is provided. When the slider 40 is disposed at a position opposite to the side of the distal end portion 23 of the inner cylinder 20 (most proximal end side), the additional ligation ring 562 is disposed at a position corresponding to the projection 26 in the axial direction. Additionally, in the axial direction, the ligation rings 60 and 61 are disposed at a position opposite to the side of the distal end portion 24 of the inner cylinder 20 with respect to the projection 26.

When the number of the ligation rings is the same, the ligation device 501 according to the third modification example can decrease an axial-direction length of the slider 540, and consequently a size of the ligation device 501 can be decreased. When the ligation device 501 is operated in vivo, an unnecessary force is applied to the ligation ring 562 from another device or a human body tissue, and failures such as erroneous ejection of the ligation ring 562 can be prevented by the slider extension portion 548.

The ligation device 501 having such a configuration can also exhibit the same effect as of the ligation device 1 according to the first embodiment. Incidentally, in the third modification example, the slider 540 need not have the slider extension portion 548.

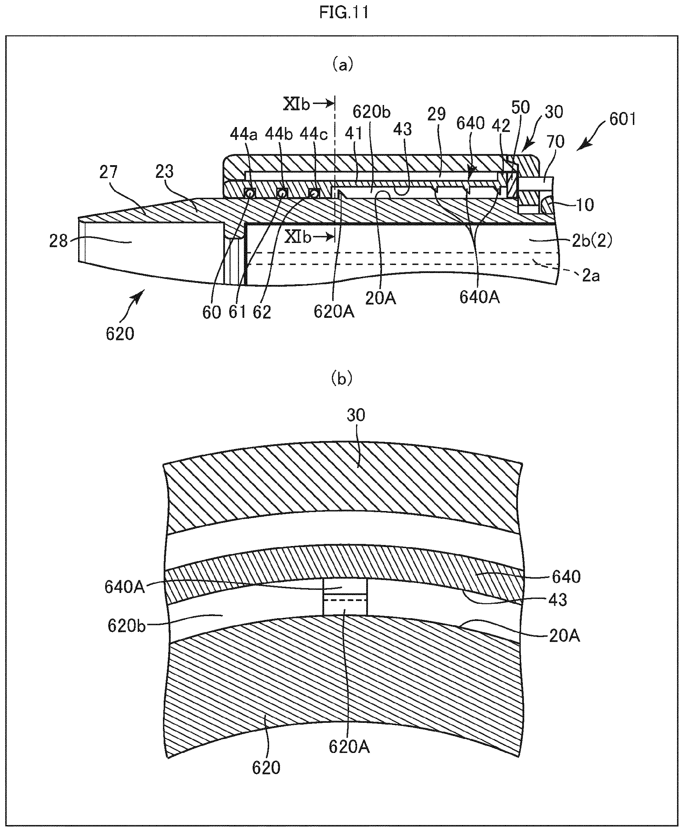

FIG. 11 (a) is a partial sectional view of a ligation device 601 according to the fourth modification example attached to the endoscope 2, illustrating a state that a slider 640 is located on a proximal end side. FIG. 11 (b) is a partial sectional view taken along line XIb-XIb in FIG. 11 (a). FIG. 11 (a) illustrates only a part of the distal end portion of the endoscope 2 equipped with the ligation device 601. In FIG. 11 (b), the endoscope 2 is omitted.

Additionally, in FIG. 11 (a), the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 601 according to the fourth modification example, the same members as in the ligation device 1 according to the first embodiment are given the same reference numerals as in the first embodiment, and detailed explanation of the same members is omitted.

The ligation device 601 includes the joining member 10, an inner cylinder 620, the outer cylinder 30, a slider 640, the sealing member 50, the ligation rings 60, 61 and 62, and the tube 70.

In the inner peripheral face 43 of the slider 640, a part of the proximal end side of the annular groove 44c has a larger diameter. An inner gap 620b is formed between the enlarged-diameter part of the inner peripheral face 43 of the slider 640 and the outer peripheral face 20A of the inner cylinder 620.

On the outer peripheral face 20A of the inner cylinder 620, a first projection 620A projecting into the inner gap 620b is placed. On the inner peripheral face 43 of the slider 640, a plurality of (three) second projections 640A projecting into the inner gap 620b are placed along the axial direction. The plurality of second projections 640A are located on the proximal end side of the first projection 620A in a state that the slider 640 is located on the proximal end side. The first projection 620A and each second projection 640A have a saw-like sectional shape obtained by cutting the slider 640 along a plane including the axial direction of the slider 640. Incidentally, the sectional shape of the projections may be a trapezoid, a chevron shape, and a cylindrical shape, or the like. The first projection 620A and each second projection 640A have a rectangular shape when viewed from the axial direction. The first projection 620A and the plurality of second projections 640A are arranged in a row along the axial direction.

The plurality of second projections 640A are configured such that a distance between the second projections 640A adjacent to each other is substantially equal to the distance between the ligation rings 60, 61 and 62 adjacent to each other. A distance between the first projection 620A and the second projection 640A located on the most distal end side is set to a distance at which the ligation ring 60 is ejected from the slider 640 when the slider 640 advances and the second projection 640A located on the most distal end side crosses over the first projection 620A.

A sum of heights of the first projection 620A and each second projection 640A in the radial direction of the slider 640 is structurally larger than a radial-direction width of the inner gap 620b (distance between the inner peripheral face 43 of the part with the enlarged diameter and the outer peripheral face 20A). That means, as illustrated in FIG. 11 (b), the first projection 620A and each second projection 640A share a part where they overlap each other when the ligation device 601 is viewed from the distal end side.

As illustrated in FIG. 11 (a), a radial-direction height of one of the plurality of second projections 640A is structurally different from radial-direction heights of the other remaining second projections 640A. In the fourth modification example, the plurality of second projections 640A are configured such that their radial-direction heights gradually increase toward the proximal end side in the axial direction. The heights of the plurality of second projections may be equal to each other.

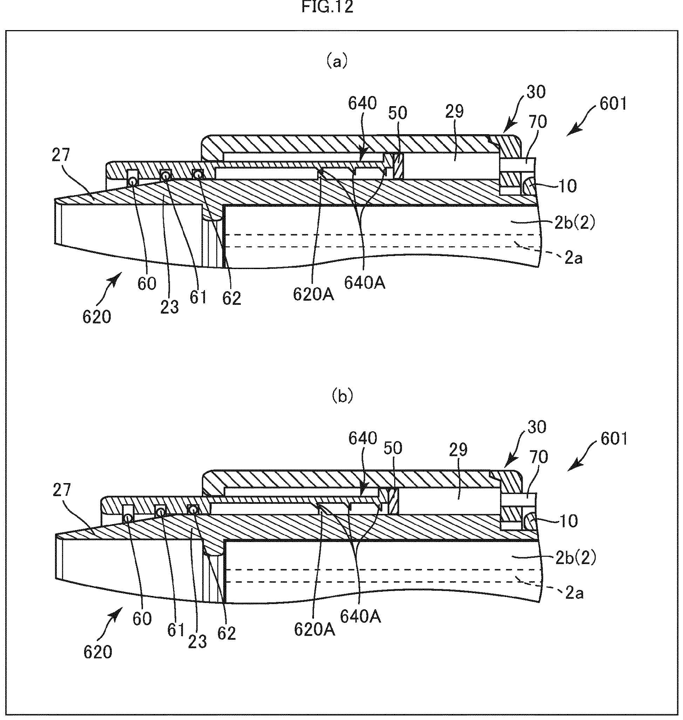

FIG. 12 (a) and FIG. 12 (b) are diagrams explaining operations of the ligation device 601.

A fluid is delivered to the slide space 29 through the tube 70 by operating a syringe not illustrated, and the slider 640 and the sealing member 50 are advanced using a pressure of the fluid as a driving source. As illustrated in FIG. 12 (a), the second projection 640A located on the most distal end side is made to abut on the first projection 620A. When the driving force of the fluid for moving the slider 640 exceeds a predetermined magnitude, the second projection 640A crosses over the first projection 620A and moves to the distal end side, as illustrated in FIG. 12 (b). At this time, the ligation ring 60 is ejected from the slider 640, moves to the distal end side on the tapered portion 27 while the diameter of the ligation ring 60 gradually decreases, and then the ligation ring 60 is ejected from the tapered portion 27. Furthermore, when the second projection 640A located in the middle in the axial direction crosses over the first projection 620A by advancing the slider 640, the ligation ring 61 is ejected from the slider 640, and when the second projection 640A located on the most proximal end side crosses over the first projection 620A, the ligation ring 62 is ejected from the slider 640.

The ligation device 601 according to the fourth modification example is configured such that a sum of heights of the first projection 620A and each second projection 640A in the radial direction of the slider 640 is larger than a radial-direction width of the inner gap 620b. Thus, for example when the second projection 640A crosses over the first projection 620A by advancing the slider 640, the ligation ring 60 is ejected from the slider 640, and this configuration makes it possible to eject the ligation ring 60 at an intended timing.

Since the plurality of second projections 640A are placed along the axial direction, the plurality of ligation rings 60, 61 and 62 can be sequentially ejected at an intended timing. A radial-direction height of one of the plurality of second projections 640A is structurally different from radial-direction heights of the other remaining second projections 640A. Thereby, a driving force for moving the slider 640 in ejecting the ligation rings 60, 61 and 62 can be changed.

The plurality of second projections 640A are configured such that their radial-direction heights gradually increase toward the proximal end side in the axial direction. Thus, for example, it is possible to prevent an event that, after the second projection 640A located on the most distal end side crosses over the first projection 620A and the ligation ring 60 is ejected, the slider 640 advances, the second projection 640A located in the middle crosses over the first projection 620A, and the ligation ring 61 is erroneously ejected.

Furthermore, the ligation device 601 having such a configuration can also exhibit the same effect as of the ligation device 1 according to the first embodiment.

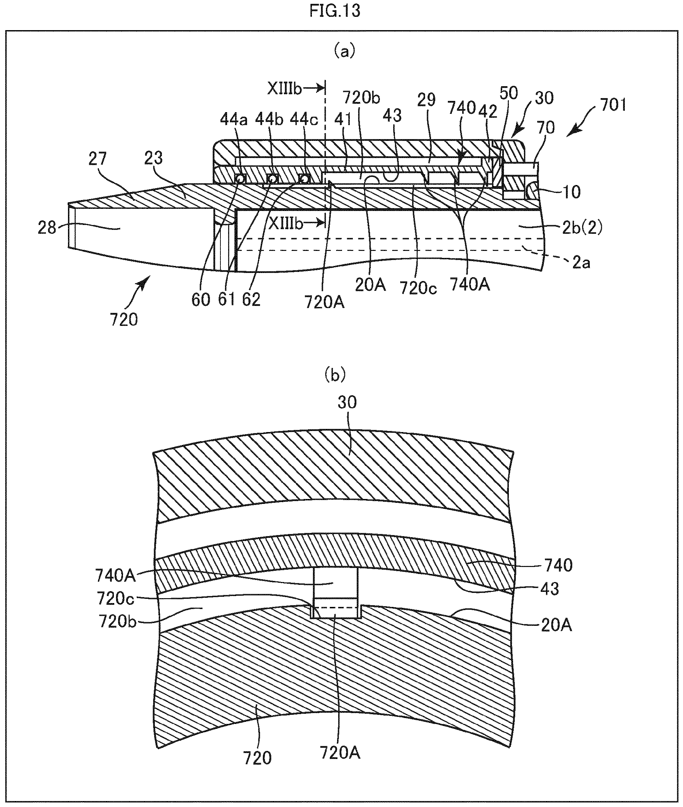

FIG. 13 (a) is a partial sectional view of a ligation device 701 according to the fifth modification example attached to the endoscope 2, illustrating a state that a slider 740 is located on a proximal end side. FIG. 13 (b) is a partial sectional view taken along line XIIIb-XIIIb in FIG. 13 (a). FIG. 13 (a) illustrates only a part of the distal end portion of the endoscope 2 equipped with the ligation device 701. In FIG. 13 (b), the endoscope 2 is omitted.

Additionally, in FIG. 13 (a), the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 701 according to the fifth modification example, the same members as in the ligation device 1 according to the first embodiment are given the same reference numerals as in the first embodiment, and detailed explanation of the same members is omitted.

The ligation device 701 includes the joining member 10, an inner cylinder 720, the outer cylinder 30, a slider 740, the sealing member 50, the ligation rings 60, 61 and 62, and the tube 70.

In the inner peripheral face 43 of the slider 740, a part of the proximal end side of the annular groove 44c has a larger diameter. An inner gap 720b is formed between the enlarged-diameter part of the inner peripheral face 43 of the slider 740 and the outer peripheral face 20A of the inner cylinder 720.

On the outer peripheral face 20A of the inner cylinder 720, a first groove 720c extending along the axial direction is formed. On a bottom portion of the first groove 720c, a first projection 720A projecting into the inner gap 720b is placed. On the inner peripheral face 43 of the slider 740, a plurality of second projections 740A projecting into the inner gap 720b are placed along the axial direction. Configurations and positional relationships of the first projection 720A and the plurality of second projections 740A are almost the same as those of the first projection 620A and the plurality of second projections 640A according to the fourth modification example. A top portion of each second projection 740A is accommodated in the first groove 720c. That means, a height of each second projection 740A is larger than a radial-direction width of the slider 740 in the inner gap 720b. The axial-direction length of the first groove 720c structurally allows the second projection 740A located on the most distal end side to be accommodated in the first groove 720c even after the second projection 740A crosses over the first projection 720A.

In the ligation device 701 according to the fifth modification example, the first projection 720A projects from the bottom portion of the first groove 720c toward the inner gap 720b, and the top portion of each second projection 740A is accommodated in the first groove 720c. Thus, the slider 740 can be prevented from rotating in the circumferential direction with respect to the inner cylinder 720. Thereby, the first projection 720A and each second projection 740A can be securely brought into contact with each other.

The ligation device 701 having such a configuration can also exhibit the same effect as of the ligation device 1 according to the first embodiment and the ligation device 601 according to the fourth modification example.

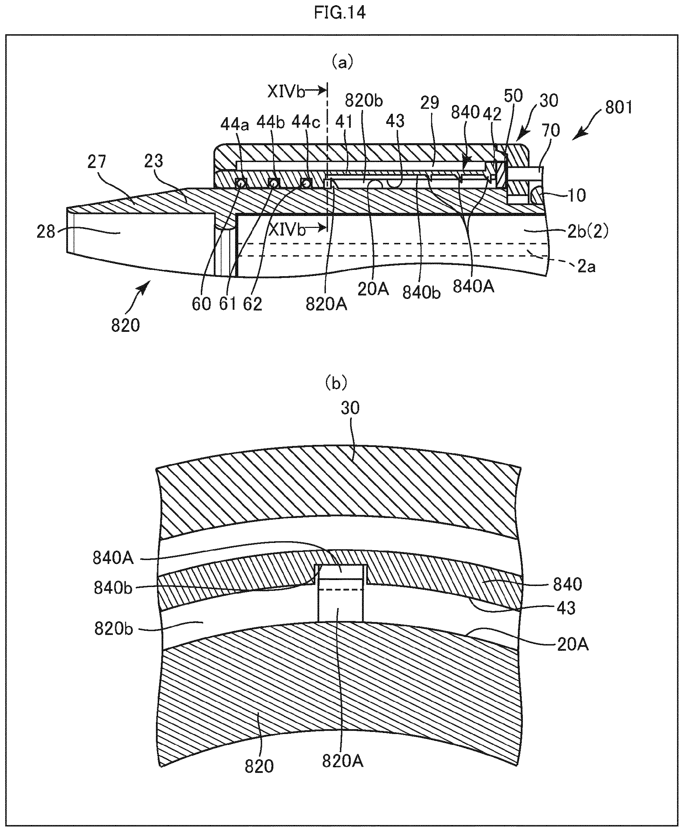

FIG. 14 (a) is a partial sectional view of a ligation device 801 according to the sixth modification example attached to the endoscope 2, illustrating a state that a slider 840 is located on a proximal end side. FIG. 14 (b) is a partial sectional view taken along line XIVb-XIVb in FIG. 14 (a). FIG. 14 (a) illustrates only a part of the distal end portion of the endoscope 2 equipped with the ligation device 801. In FIG. 14 (b), the endoscope 2 is omitted.

Additionally, in FIG. 14 (a), the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 801 according to the sixth modification example, the same members as in the ligation device 1 according to the first embodiment are given the same reference numerals as in the first embodiment, and detailed explanation of the same members is omitted.

The ligation device 801 includes the joining member 10, an inner cylinder 820, the outer cylinder 30, a slider 840, the sealing member 50, the ligation rings 60, 61 and 62, and the tube 70.

In the inner peripheral face 43 of the slider 840, a part of the proximal end side of the annular groove 44c has a larger diameter. An inner gap 820b is formed between the enlarged-diameter part of the inner peripheral face 43 of the slider 840 and the outer peripheral face 20A of the inner cylinder 820.

On the outer peripheral face 20A of the inner cylinder 820, a first projection 820A projecting into the inner gap 820b is placed. On the inner peripheral face 43 of the slider 840, a second groove 840b extending along the axial direction is formed. On the bottom portion of the second groove 840b, a plurality of second projections 840A projecting into the inner gap 820b are placed along the axial direction. Configurations and positional relationships of the first projection 820A and the plurality of second projections 840A are almost the same as those of the first projection 620A and the plurality of second projections 640A according to the fourth modification example. A top portion of the first projection 820A is accommodated in the second groove 840b. That means, a height of the first projection 820A is larger than a radial-direction width of the slider 840 in the inner gap 820b.

In the ligation device 801 according to the sixth modification example, each second projection 820A projects from the bottom portion of the second groove 840b toward the inner gap 820b, and the top portion of the first projection 820A is accommodated in the second groove 820b. Thus, the slider 840 can be prevented from rotating in the circumferential direction with respect to the inner cylinder 820. Thereby, the first projection 820A and each second projection 840A can be securely brought into contact with each other.

The ligation device 801 having such a configuration can also exhibit the same effect as of the ligation device 1 according to the first embodiment and the ligation device 601 according to the fourth modification example.

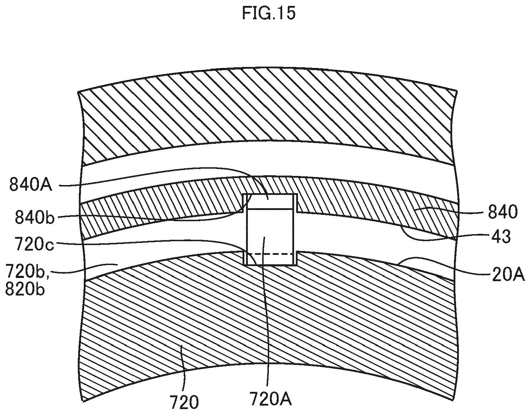

In addition, as illustrated in FIG. 15, the inner cylinder 720 according to the fifth modification example and the slider 840 according to the sixth modification example may be combined. That means, the top portion of the first projection 720A placed on the bottom portion of the first groove 720c may be accommodated in the second groove 840b, and the top portion of each second projection 840A placed on the bottom portion of the second groove 840b may be accommodated in the first groove 720c.

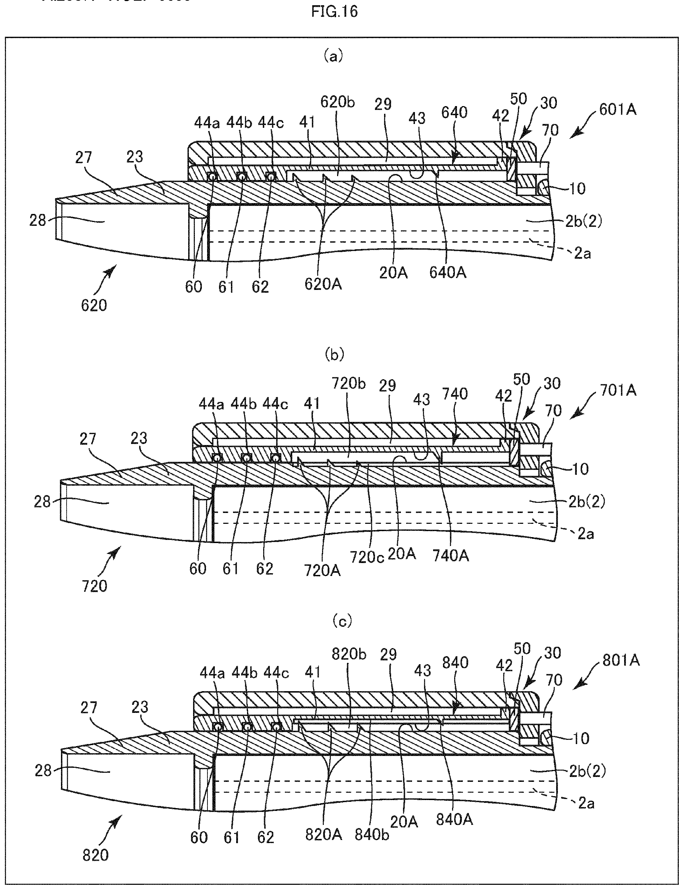

FIG. 16 (a) is a partial sectional view of a ligation device 601A according to a modification example of the fourth modification example (FIG. 11) attached to the endoscope 2, illustrating a state that the slider 640 is located on the proximal end side.

In the fourth modification example illustrated in FIG. 11, one first projection 620A projecting into the inner gap 620b is placed on the outer peripheral face 20A of the inner cylinder 620, and the plurality of second projections 640A projecting into the inner gap 620b are placed along the axial direction on the inner peripheral face 43 of the slider 640. On the other hand, in the modification example of the fourth modification example, a plurality of the first projections 620A projecting into the inner gap 620b are placed along the axial direction on the outer peripheral face 20A of the inner cylinder 620, and one piece of second projection 640A projecting into the inner gap 620b is placed on the inner peripheral face 43 of the slider 640, as illustrated in FIG. 16 (a).

The plurality of first projections 620A are located on the distal end side of the second projection 640A in a state that the slider 640 is located on the proximal end side. The plurality of first projections 620A and second projection 640A are arranged in a row along the axial direction.

The plurality of first projections 620A are configured such that a distance between the first projections 620A adjacent to each other is substantially equal to the distance between the ligation rings 60, 61 and 62 adjacent to each other. A distance between the second projection 640A and the first projection 620A located on the most proximal end side is set to a distance at which the ligation ring 60 is ejected from the slider 640 when the slider 640 advances and the second projection 640A crosses over the first projection 620A located on the most proximal end side.

A sum of heights of each first projection 620A and the second projection 640A in the radial direction of the slider 640 is structurally larger than a radial-direction width of the inner gap 620b (distance between the inner peripheral face 43 of the part with the enlarged diameter and the outer peripheral face 20A).

A radial-direction height of one of the plurality of first projections 620A is structurally different from radial-direction heights of the other remaining first projections 620A. In the modification example of the fourth modification example, the plurality of first projections 620A are configured such that their radial-direction heights gradually increase toward the distal end side in the axial direction. Incidentally, the slider 640 according to the modification example of the fourth modification example is configured to have an axial-direction length larger than of the slider 640 according to the fourth modification example. The heights of the plurality of first projections may be equal to each other.

The ligation device 601A according to the modification example of the fourth modification example can also exhibit the same effect as of the ligation device 1 according to the first embodiment and the ligation device 601 according to the fourth modification example.

FIG. 16 (b) is a partial sectional view of a ligation device 701A according to a modification example of the fifth modification example (FIG. 13) attached to the endoscope 2, illustrating a state that the slider 740 is located on the proximal end side.

In the fifth modification example illustrated in FIG. 13, one first projection 720A projecting into the inner gap 720b is placed on the bottom portion of the first groove 720c, and the plurality of second projections 740A projecting into the inner gap 720b are placed along the axial direction on the inner peripheral face 43 of the slider 740. On the other hand, in the modification example of the fifth modification example, the plurality of first projections 720A projecting into the inner gap 720b are placed along the axial direction on the bottom portion of the first groove 720c, and one piece of second projection 740A projecting into the inner gap 720b is placed on the inner peripheral face 43 of the slider 740, as illustrated in FIG. 16 (b).

Configurations and positional relationships of the plurality of first projections 720A and second projection 740A are almost the same as those of the first projection 620A and the plurality of second projections 640A according to the modification example in FIG. 16 (a). A top portion of each second projection 740A is accommodated in the first groove 720c. That means, a height of each second projection 740A is larger than a radial-direction width of the slider 740 in the inner gap 720b. Incidentally, the slider 740 according to the modification example of the fifth modification example is configured to have an axial-direction length larger than of the slider 740 according to the fifth modification example.

The ligation device 701A according to the modification example of the fifth modification example can also exhibit the same effect as of the ligation device 1 according to the first embodiment and the ligation device 701 according to the fifth modification example.

FIG. 16 (c) is a partial sectional view of a ligation device 801A according to a modification example of the sixth modification example (FIG. 14) attached to the endoscope 2, illustrating a state that the slider 840 is located on the proximal end side.

In the sixth modification example illustrated in FIG. 14, one first projection 820A projecting into the inner gap 820b is placed on the outer peripheral face 20A of the inner cylinder 820, and the plurality of second projections 840A projecting into the inner gap 820b are placed along the axial direction on the bottom portion of the second groove 840b. On the other hand, in the modification example of the sixth modification example, the plurality of first projections 820A projecting into the inner gap 820b are placed along the axial direction on the outer peripheral face 20A of the inner cylinder 820, and one piece of second projection 840A projecting into the inner gap 820b is placed on the bottom portion of the second groove 840b, as illustrated in FIG. 16 (c).

Configurations and positional relationships of the plurality of first projections 820A and second projection 840A are almost the same as those of the first projection 620A and the plurality of second projections 640A according to the modification example in FIG. 16 (a). Top portions of the plurality of first projections 820A are accommodated in the second groove 840b. That means, heights of the plurality of first projections 820A are larger than a radial-direction width of the slider 840 in the inner gap 820b. Incidentally, the slider 840 according to the modification example of the sixth modification example is configured to have an axial-direction length larger than of the slider 840 according to the sixth modification example.

The ligation device 801A according to the modification example of the sixth modification example can also exhibit the same effect as of the ligation device 1 according to the first embodiment and the ligation device 801 according to the sixth modification example.

Incidentally, in the aforementioned modification examples, each of the first projections 620A, 720A and 820A may have an annular shape extending in the circumferential directions of the inner cylinders 620, 720 and 820 respectively. Each of the second projections 640A, 740A and 840A may have an annular shape extending in the circumferential directions of the sliders 640, 740 and 840 respectively. Both of each of first projections 620A, 720A and 820A and each of the second projections 640A, 740A and 840A may have an annular shape extending in the circumferential directions of the inner cylinders 620, 720 and 820 and the sliders 640, 740 and 840 respectively.

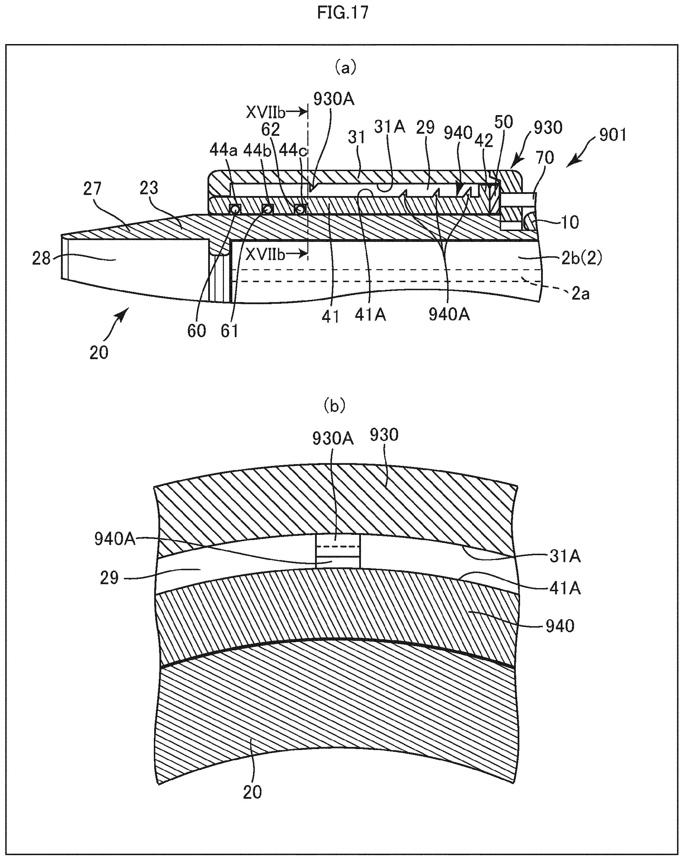

FIG. 17 (a) is a partial sectional view of a ligation device 901 according to the seventh modification example attached to the endoscope 2, illustrating a state that a slider 940 is located on a proximal end side. FIG. 17 (b) is a partial sectional view taken along line XVIIb-XVIIb in FIG. 17 (a). FIG. 17 (a) illustrates only a part of the distal end portion of the endoscope 2 equipped with the ligation device 901. In FIG. 17 (b), the endoscope 2 is omitted.

Additionally, in FIG. 17 (a), the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 901 according to the seventh modification example, the same members as in the ligation device 1 according to the first embodiment are given the same reference numerals as in the first embodiment, and detailed explanation of the same members is omitted.

The ligation device 901 includes the joining member 10, the inner cylinder 20, an outer cylinder 930, a slider 940, the sealing member 50, the ligation rings 60, 61 and 62, and the tube 70.

On an outer peripheral face 41A of the cylindrical portion 41 of the slider 940, a plurality of (three) third projections 940A projecting into the slide space 29 are placed along the axial direction. On an inner peripheral face 31A of the outer cylindrical portion 31 of the outer cylinder 30, a fourth projection 930A projecting into the slide space 29 is placed. The slide space 29 corresponds to the outer gap.

The plurality of third projections 940A are located on the proximal end side of the fourth projection 930A in a state that the slider 940 is located on the proximal end side. Each third projection 940A and the fourth projection 930A have a saw-like sectional shape obtained by cutting the slider 640 along a plane including the axial direction of the slider 640. Incidentally, the shape of the projections may be a trapezoid, a chevron shape, and a cylindrical shape, or the like. Each third projection 940A and the fourth projection 930A have a rectangular shape when viewed from the axial direction. The plurality of third projections 940A and the fourth projection 930A are arranged in a row along the axial direction.

The plurality of third projections 940A are configured such that a distance between the third projections 940A adjacent to each other is substantially equal to the distance between the ligation rings 60, 61 and 62 adjacent to each other. A distance between the fourth projection 930A and the third projection 940A located on the most distal end side is set to a distance at which the ligation ring 60 is ejected from the slider 940 when the slider 940 advances and the third projection 940A located on the most distal end side crosses over the fourth projection 930A.

A sum of heights of each third projection 940A and the fourth projection 930A in the radial direction of the slider 940 is structurally larger than a radial-direction width of the slide space 29 (distance between the outer peripheral face 41A and the inner peripheral face 31A. That means, as illustrated in FIG. 17 (b), each third projection 940A and the fourth projection 930A share a part where they overlap each other when the ligation device 901 is viewed from the distal end side.

As illustrated in FIG. 17 (a), a radial-direction height of one of the plurality of third projections 940A is structurally different from radial-direction heights of the other remaining third projections 940A. In the seventh modification example, the plurality of third projections 940A are configured such that their radial-direction heights gradually increase toward the proximal end side in the axial direction. The heights of the plurality of third projections may be equal to each other.

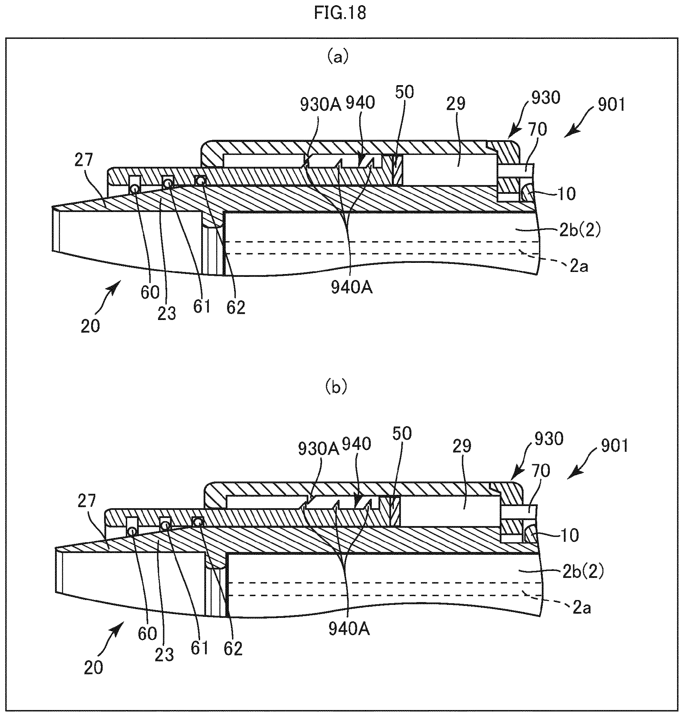

FIG. 18 (a) and FIG. 18 (b) are diagrams explaining operations of the ligation device 901.

A fluid is delivered to the slide space 29 through the tube 70 by operating a syringe not illustrated, and the slider 940 and the sealing member 50 are advanced using a pressure of the fluid as a driving source. As illustrated in FIG. 18 (a), the third projection 940A located on the most distal end side is made to abut on the fourth projection 930A. When the driving force of the fluid for moving the slider 940 exceeds a predetermined magnitude, the third projection 940A crosses over the fourth projection 930A and move to the distal end side, as illustrated in FIG. 18 (b). At this time, the ligation ring 60 is ejected from the slider 940, moves to the distal end side on the tapered portion 27 while the diameter of the ligation ring 60 gradually decreases, and then the ligation ring 60 is ejected from the tapered portion 27. Furthermore, when the third projection 940A located in the middle in the axial direction crosses over the fourth projection 930A by advancing the slider 940, the ligation ring 61 is ejected from the slider 940, and when the third projection 940A located on the most proximal end side crosses over the fourth projection 930A, the ligation ring 62 is ejected from the slider 940.

The ligation device 901 according to the seventh modification example is configured such that a sum of heights of each third projection 940A and the fourth projection 930A in the radial direction of the slider 940 is larger than a radial-direction width of the slide space 29. Thus, for example, when the third projection 940A crosses over the ninth projection 930A by advancing the slider 940, the ligation ring 60 is ejected from the slider 940, and this configuration makes it possible to eject the ligation ring 60 at an intended timing.

Since the plurality of third projections 940A are placed along the axial direction, the plurality of ligation rings 60, 61 and 62 can be sequentially ejected at an intended timing. A radial-direction height of one of the plurality of third projections 940A is structurally different from radial-direction heights of the other remaining third projections 940A. Thereby, a driving force for moving the slider 940 in ejecting the ligation rings 60, 61 and 62 can be changed.

The plurality of third projections 940A are configured such that their radial-direction heights gradually increase toward the proximal end side in the axial direction. Thus, for example, it is possible to prevent an event that, after the third portion 940A located on the most distal end side crosses over the fourth projection 930A and the ligation ring 60 is ejected, the slider 940 advances, the third projection 940A located in the middle crosses over the fourth projection 930A, and the ligation ring 61 is erroneously ejected.

Furthermore, the ligation device 901 having such a configuration can also exhibit the same effect as of the ligation device 1 according to the first embodiment.

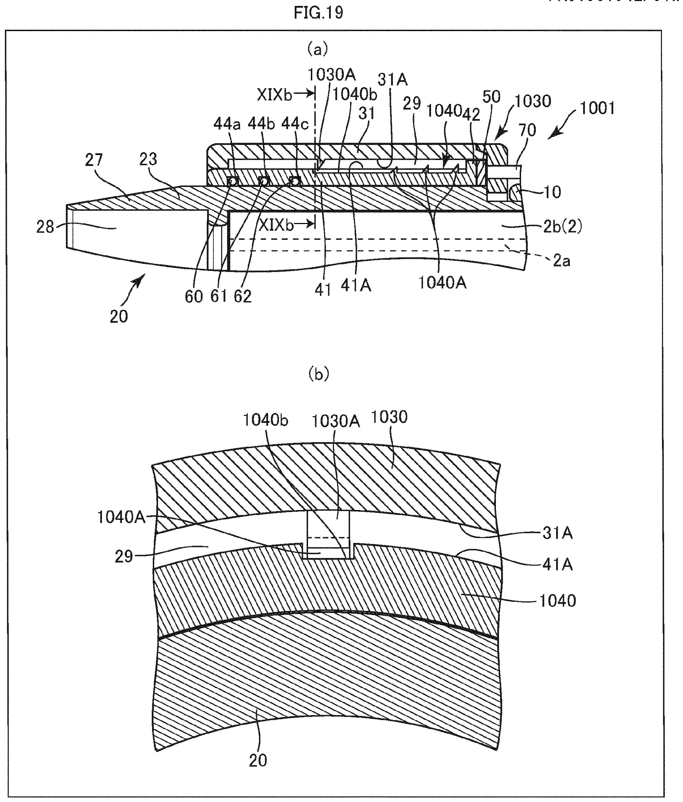

FIG. 19 (a) is a partial sectional view of a ligation device 1001 according to the eighth modification example attached to the endoscope 2, illustrating a state that a slider 1040 is located on a proximal end side. FIG. 19 (b) is a partial sectional view taken along line XIXb-XIXb in FIG. 19 (a). FIG. 19 (a) illustrates only a part of the distal end portion of the endoscope 2 equipped with the ligation device 1001. In FIG. 19 (b), the endoscope 2 is omitted.

Additionally, in FIG. 19 (a), the left side of the figure is a distal end side (farther side) to be inserted into a body, and the right side is a proximal end side (hand side, nearer side). The endoscope 2 has the forceps hole 2a through which forceps not illustrated are inserted.

Note that, in the ligation device 1001 according to the eighth modification example, the same members as in the ligation device 1 according to the first embodiment are given the same reference numerals as in the first embodiment, and detailed explanation of the same members is omitted.

The ligation device 1001 includes the joining member 10, the inner cylinder 20, an outer cylinder 1030, a slider 1040, the sealing member 50, the ligation rings 60, 61 and 62, and the tube 70.

On the outer peripheral face 41A of the cylindrical portion 41 of the slider 1040, a third groove 1040b extending the axial direction is formed. On a bottom portion of the third groove 1040b, a plurality of third projections 1040A projecting into the slide space 29 are placed along the axial direction. On the inner peripheral face 31A of the outer cylindrical portion 31 of the outer cylinder 1030, a fourth projection 1030A projecting into the slide space 29 is placed. Configurations and positional relationships of the plurality of third projections 1040A and the fourth projection 1030A are almost the same as those of the plurality of third projections 1040A and the fourth projection 1030A according to the seventh modification example. A top portion of the fourth projection 1030A is accommodated in the third groove 1040b. That means, a height of the fourth projection 1030A is larger than a radial-direction width of the slider 1040 in the slide space 29.

In the ligation device 1001 according to the eighth modification example, the plurality of third projections 1040A project from the bottom portion of the third groove 1040b toward the slide space 29, and the top portion of the fourth projection 1030A is accommodated in the third groove 1040b. Thus, the slider 1040 can be prevented from rotating in the circumferential direction with respect to the outer cylinder 1030. Thereby, each third projection 1040A and the fourth projection 1030A can be securely brought into contact with each other.

The ligation device 1001 having such a configuration can also exhibit the same effect as of the ligation device 1 according to the first embodiment and the ligation device 901 according to the seventh modification example.

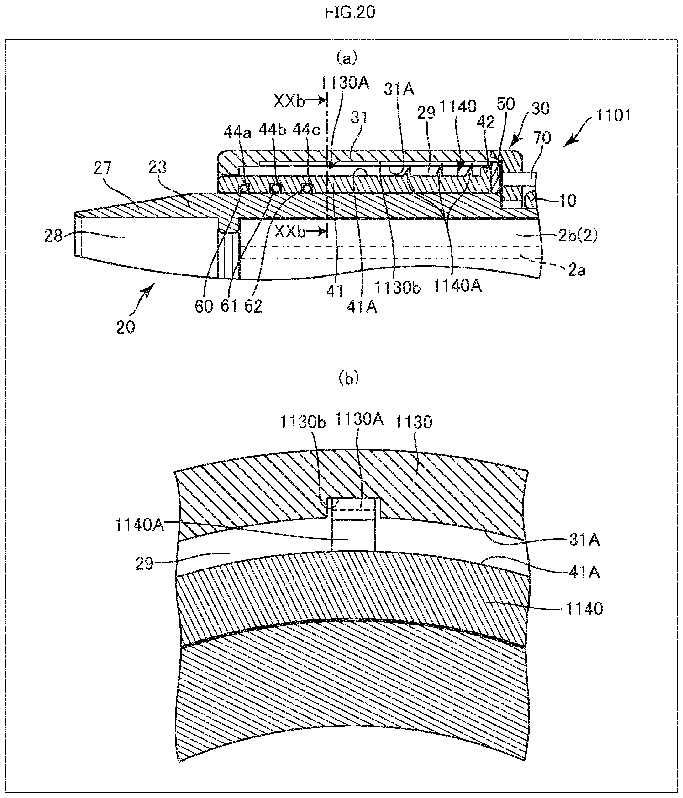

FIG. 20 (a) is a partial sectional view of a ligation device 1101 according to the ninth modification example attached to the endoscope 2, illustrating a state that a slider 1140 is located on a proximal end side. FIG. 20 (b) is a partial sectional view taken along line XXb-XXb in FIG. 20 (a). FIG. 20 (a) illustrates only a part of the distal end portion of the endoscope 2 equipped with the ligation device 1101. In FIG. 20 (b), the endoscope 2 is omitted.

Additionally, in FIG. 20 (a), the left side of the figure is a distal end side (farther side) to be inserted into a body, and the right side is a proximal end side (hand side, nearer side). The endoscope 2 has the forceps hole 2a through which forceps not illustrated are inserted.

Incidentally, in the ligation device 1101 according to the ninth modification example, the same members as in the ligation device 1 according to the first embodiment are given the same reference numerals as in the first embodiment, and detailed explanation of the same members is omitted.

The ligation device 1101 includes the joining member 10, the inner cylinder 20, an outer cylinder 1130, the slider 1140, the sealing member 50, the ligation rings 60, 61 and 62, and the tube 70.

On the outer peripheral face 41A of the cylindrical portion 41 of the slider 1140, a plurality of third projections 1140A projecting into the slide space 29 are placed. On the inner peripheral face 31A of the outer cylindrical portion 31 of the outer cylinder 30, a fourth groove 1130b extending along the axial direction is formed. On the bottom portion of the fourth groove 1130b, a fourth projections 1130A projecting into the slide space 29 is placed. Configurations and positional relationships of the plurality of third projections 1140A and the fourth projection 1130A are almost the same as those of the plurality of third projections 1040A and the fourth projection 1030A according to the seventh modification example. A top portion of each third projection 1140A is accommodated in the fourth groove 1130b. That means, a height of each third projection 1140A is larger than a radial-direction width of the slider 1140 in the slide space 29.

In the ligation device 1101 according to the ninth modification example, the fourth projection 1130A projects from the bottom portion of the fourth groove 1130b toward the slide space 29, and the top portion of each third projection 1140A is accommodated in the fourth groove 1130b. Thus, the slider 1140 can be prevented from rotating in the circumferential direction with respect to the outer cylinder 1130. Thereby, each third projection 1140A and each fourth projection 1130A can be securely brought into contact with each other.

The ligation device 1101 having such a configuration can also exhibit the same effect as of the ligation device 1 according to the first embodiment and the ligation device 901 according to the seventh modification example.

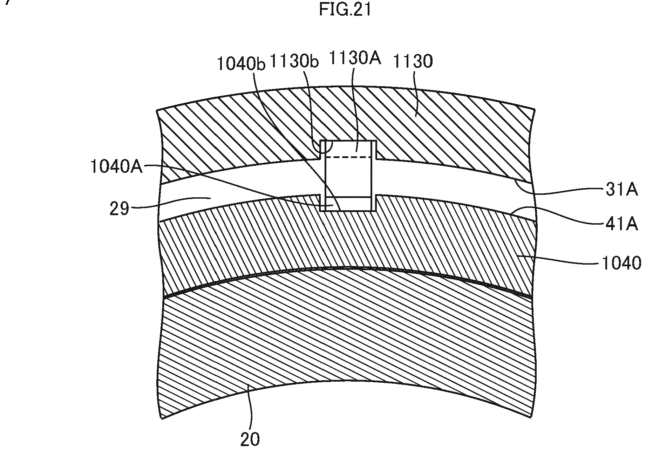

In addition, as illustrated in FIG. 21, the slider 1040 according to the eighth modification example and the outer cylinder 1130 according to the ninth modification example may be combined. That means, the top portion of each third projection 1040A placed on the bottom portion of the third groove 1040b may be accommodated in the fourth groove 1130b, and the top portion of the fourth projection 1130A placed on the bottom portion of the fourth groove 1130b may be accommodated in the third groove 1040b.

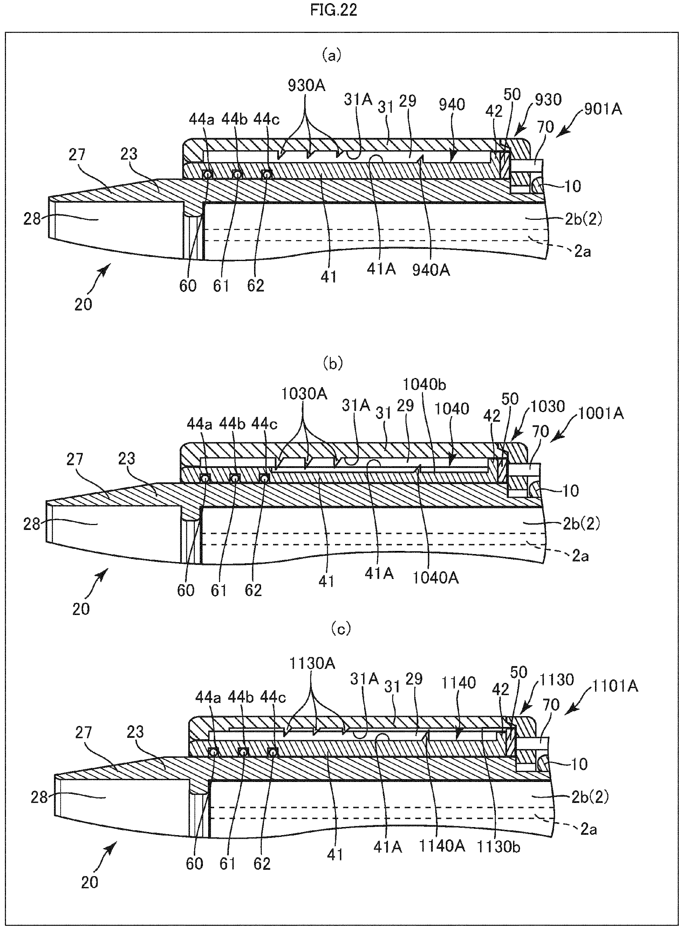

FIG. 22 (a) is a partial sectional view of a ligation device 901A according to a modification example of the seventh modification example (FIG. 17) attached to the endoscope 2, illustrating a state that the slider 940 is located on the proximal end side.

In the seventh modification example illustrated in FIG. 17, the plurality of third projections 940A projecting into the slide space 29 are placed along the axial direction on the outer peripheral face 41A of the slider 940, and the fourth projection 930A projecting into the slide space 29 is placed on the inner peripheral face 31A of the outer cylinder 30. On the other hand, in the modification example of the seventh modification example, one piece of third projection 940A projecting into the slide space 29 is placed on the outer peripheral face 41A of the slider 940, and the plurality of fourth projections 930A projecting into the slide space 29 are placed along the axial direction on the inner peripheral face 31A of the outer cylinder 30, as illustrated in FIG. 22 (a).

The plurality of fourth projections 930A are located on the distal end side of the third projection 940A in a state that the slider 940 is located on the proximal end side. The third projection 940A and the plurality of fourth projections 930A are arranged in a row along the axial direction.

The plurality of fourth projections 930A are configured such that a distance between the fourth projections 930A adjacent to each other is substantially equal to the distance between the ligation rings 60, 61 and 62 adjacent to each other. A distance between the third projection 940A and the fourth projection 930A located on the most proximal end side is set to a distance at which the ligation ring 60 is ejected from the slider 940 when the slider 940 advances and the third projection 940A crosses over the fourth projection 930A located on the most proximal end side.

A sum of heights of the third projection 940A and each fourth projection 930A in the radial direction of the slider 940 is structurally larger than the radial-direction width of the slide space 29 (distance between the outer peripheral face 41A and the inner peripheral face 31A).

A radial-direction height of one of the plurality of fourth projections 930A is structurally different from radial-direction heights of the other remaining fourth projections 930A. In the modification example of the seventh modification example, the plurality of fourth projections 930A are configured such that their radial-direction heights gradually increase toward the distal end side in the axial direction. Incidentally, the slider 940 according to the modification example of the seventh modification example is configured to have an axial-direction length larger than of the slider 940 according to the seventh modification example. The heights of the plurality of fourth projections may be equal to each other.

The ligation device 901A according to the modification example of the seventh modification example can also exhibit the same effect as of the ligation device 1 according to the first embodiment and the ligation device 901 according to the seventh modification example.

FIG. 22 (b) is a partial sectional view of a ligation device 1001A according to a modification example of the eighth modification example (FIG. 19) attached to the endoscope 2, illustrating a state that the slider 1040 is located on the proximal end side.

In the eighth modification example illustrated in FIG. 19, the plurality of third projections 1040A projecting into the slide space 29 are placed along the axial direction on the bottom portion of the third groove 1040b, and one piece of fourth projection 1030A projecting into the slide space 29 is placed on the inner peripheral face 31A of the outer cylinder 1030. On the other hand, in the modification example of the eighth modification example, one piece of third projection 1040A projecting into the slide space 29 is placed on the bottom portion of the third groove 1040b, and the plurality of fourth projections 1030A projecting into the slide space 29 are placed along the axial direction on the inner peripheral face 31A of the outer cylinder 1030, as illustrated in FIG. 22 (b).

Configurations and positional relationships of the third projections 1040A and the plurality of fourth projections 1030A are almost the same as those of the third projection 1040A and the plurality of fourth projections 1030A according to the modification example in FIG. 22 (a). The top portion of each fourth projection 1030A is accommodated in the third groove 1040b. That means, the height of each fourth projection 1030A is larger than the radial-direction width of the slider 1040 in the slide space 29. Incidentally, the slider 1040 according to the modification example of the eighth modification example is configured to have an axial-direction length larger than of the slider 1040 according to the eighth modification example.

The ligation device 1001A according to the modification example of the eighth modification example can also exhibit the same effect as of the ligation device 1 according to the first embodiment and the ligation device 1001 according to the eighth modification example.

FIG. 22 (c) is a partial sectional view of a ligation device 1101A according to the modification example of the ninth modification example (FIG. 20) attached to the endoscope 2, illustrating a state that the slider 1140 is located on the proximal end side.

In the ninth modification example illustrated in FIG. 20, the plurality of third projections 1140A projecting into the slide space 29 are placed along the axial direction on the outer peripheral face 41A of the slider 1140, and one piece of fourth projection 1130A projecting into the slide space 29 is placed on the bottom portion of fourth groove 1130b. On the other hand, in the modification example of the ninth modification example, one piece of third projection 1140A projecting into the slide space 29 is placed on the outer peripheral face 41A of the slider 1140, and the plurality of fourth projections 1130A projecting into the slide space 29 are placed along the axial direction on the bottom portion of the fourth groove 1130b, as illustrated in FIG. 22 (c).

Configurations and positional relationships of the third projections 1140A and the plurality of fourth projections 1130A are almost the same as those of the third projection 1140A and the plurality of fourth projections 1130A according to the modification example in FIG. 22 (a). The top portion of each fourth projection 1130A is accommodated in the fourth groove 1130b. That means, the heights of the plurality of fourth projections 1130A are larger than the radial-direction width of the slider 1140 in the slide space 29. Incidentally, the slider 1140 according to the modification example of the ninth modification example is configured to have an axial-direction length larger than of the slider 1140 according to the ninth modification example.

The ligation device 1101A according to the modification example of the ninth modification example can also exhibit the same effect as of the ligation device 1 according to the first embodiment and the ligation device 1101 according to the sixth modification example.

Incidentally, in the aforementioned modification examples, each of the third projections 940A, 1040A and 1140A may have an annular shape extending in the circumferential directions of the sliders 940, 1040 and 1140 respectively. Each of the fourth projections 930A, 1030A and 1130A may have an annular shape extending in the circumferential directions of the outer cylinders 930, 1030 and 1130, respectively. Both of each of the third projections 940A, 1040A and 1140A and each of the fourth projections 930A, 1030A and 1130A may have an annular shape extending in the circumferential directions of the sliders 940, 1040, 1140 and the outer cylinders 930, 1030 and 1130 respectively.

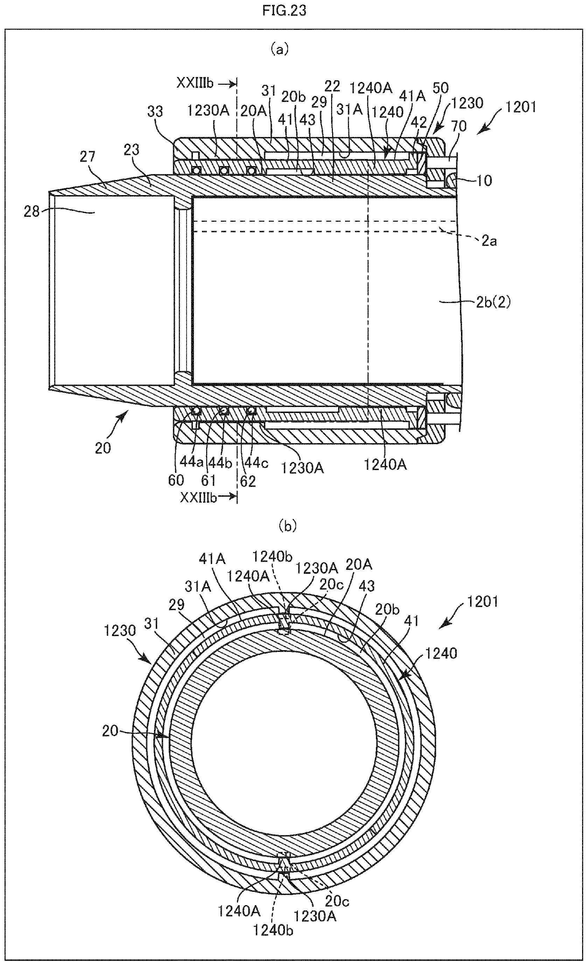

FIG. 23 (a) is a sectional view of a ligation device 1201 according to the tenth modification example attached to the endoscope 2, illustrating a state that a slider 1240 is located on a proximal end side. FIG. 23 (b) is a sectional view taken along line XXIIIb-XXIIIb in FIG. 23 (a). FIG. 23 (a) illustrates only a part of the distal end portion of the endoscope 2 equipped with the ligation device 1201. In FIG. 23 (b), the endoscope 2 is omitted.

Additionally, in FIG. 23 (a), the left side of the figure is a distal end side (farther side) to be inserted into a body, and the right side is a proximal end side (hand side, nearer side). The endoscope 2 has the forceps hole 2a through which forceps not illustrated are inserted.

Incidentally, in the ligation device 1201 according to the tenth modification example, the same members as in the ligation device 1 according to the first embodiment are given the same reference numerals as in the first embodiment, and detailed explanation of the same members is omitted.

The ligation device 1201 includes the joining member 10, the inner cylinder 20, an outer cylinder 1230, a slider 1240, the sealing member 50, the ligation rings 60, 61 and 62, and the tube 70.

In the inner peripheral face 43 of the slider 1240, a part of the proximal end side of the annular groove 44c has a larger diameter. The inner gap 20b is formed between the enlarged-diameter part of the inner peripheral face 43 of the slider 1240 and the outer peripheral face 20A of the inner cylinder 20.

On the inner peripheral face 43 of the slider 1240, a plurality of sixth projections 1240A that project into the inner gap 20b to abut on the outer peripheral face 20A of the inner cylinder 20 are placed. Each sixth projection 1240A extends along the axial direction. On the inner peripheral face 31A of the outer cylindrical portion 31 of the outer cylinder 30, a plurality of eighth projections 1230A that project into the slide space 29 to abut on the outer peripheral face 41A of the slider 1240 are placed. Each eighth projection 1230A extends along the axial direction.

According to the configuration of the ligation device 1201 according to the tenth modification example, when the slider 1240 moves in the axial direction, straightness of the slider 1240 can be improved without increasing the frictional resistance between the inner cylinder 20 and the outer cylinder 30.

The ligation device 1201 according to the tenth modification example can also exhibit the same effect as of the ligation device 1 according to the first embodiment.

Incidentally, as represented by the dashed line in FIG. 23 (b), grooves 20c and 1240b extending along the axial direction may be formed on the outer peripheral face 20A of the inner cylinder 20 and the outer peripheral face 41A of the slider 1240 respectively, so that top portions of the sixth projections 1240A and the eighth projections 1230A are accommodated in the grooves. Although the sixth projections 1240A and the eighth projections 1230A are placed on the ligation device 1201, either the sixth or eighth projections may be placed.

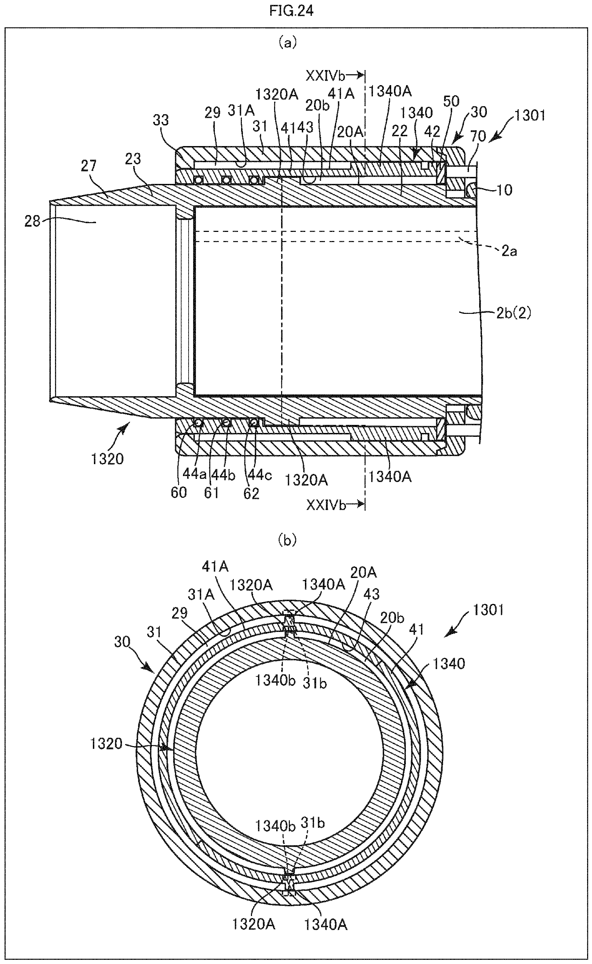

FIG. 24 (a) is a sectional view of a ligation device 1301 according to the eleventh modification example attached to the endoscope 2, illustrating a state that a slider 1340 is located on a proximal end side. FIG. 24 (b) is a sectional view taken along line XXIVb-XXIVb in FIG. 24 (a). FIG. 24 (a) illustrates only a part of the distal end portion of the endoscope 2 equipped with the ligation device 1301. In FIG. 24 (b), the endoscope 2 is omitted. Additionally, in FIG. 24 (a), the left side of the figure is a distal end side (farther side) to be inserted into a body, and the right side is a proximal end side (hand side, nearer side). The endoscope 2 has the forceps hole 2a through which forceps not illustrated are inserted.

Incidentally, in the ligation device 1301 according to the eleventh modification example, the same members as in the ligation device 1 according to the first embodiment are given the same reference numerals as in the first embodiment, and detailed explanation of the same members is omitted.

The ligation device 1301 includes the joining member 10, an inner cylinder 1320, the outer cylinder 30, a slider 1340, the sealing member 50, the ligation rings 60, 61 and 62, and the tube 70.

In the inner peripheral face 43 of the slider 1340, a part of the proximal end side of the annular groove 44c has a larger diameter. The inner gap 20b is formed between the enlarged-diameter part of the inner peripheral face 43 of the slider 1240 and the outer peripheral face 20A of the inner cylinder 20.

On the outer peripheral face 20A of the inner cylinder 1320, a plurality of fifth projections 1320A that project into the inner gap 20b to abut on the inner peripheral face 43 of the slider 1340 are placed. Each fifth projection 1320A extends along the axial direction. On the outer peripheral face 41A of the slider 1240, a plurality of seventh projections 1340A that project into the slide space 29 to abut on the inner peripheral face 31A of the outer cylinder 30 are placed. Each seventh projection 1340A extends along the axial direction.

According to the configuration of the ligation device 1301 according to the eleventh modification example, when the slider 1240 moves in the axial direction, straightness of the slider 1240 can be improved without increasing the frictional resistance between the inner cylinder 20 and the outer cylinder 30.

The ligation device 1301 according to the eleventh modification example can also exhibit the same effect as of the ligation device 1 according to the first embodiment.

As represented by the dashed line in FIG. 24 (b), grooves 1340b and 31b extending along the axial direction may be formed on the inner peripheral face 43 of the slider 1340 and the inner peripheral face 31A of the outer cylinder 30 respectively, so that top portions of the fifth projections 1320A and the seventh projections 1340A are accommodated in the grooves. Although the fifth projections 1320A and the seventh projections 1340A are placed on the ligation device 1301, either the fifth or seventh projections may be placed. In the ligation device 1301, the sixth projections 1240A and eighth projections 1230A according to the tenth modification example may be placed in addition to the fifth projections 1320A and the seventh projections 1340A.

### DESCRIPTION OF REFERENCE NUMERALS

1, 101, 201, 301, 401, 501, 601, 601A, 701, 701A, 801, 801A, 901, 901A, 1001, 1001A, 1101, 1101A, 1201, 1301 Ligation device
2 Endoscope
2b Endoscope distal end portion
20, 120, 620, 720, 820, 1320 Inner cylinder
23, 123, 223 Distal end portion
27,227 Tapered portion
29 Slide space
30, 930, 1030, 1130, 1230 Outer cylinder
40, 540, 640, 740, 840, 940, 1040, 1140, 1240, 1340 Slider
43 Inner peripheral face
44a, 44b, 44c Annular groove
44d Bottom face
45, 46, 145, 146 Slider piece
60, 61, 62, 63 Ligation ring
548 Slider extension portion
562 Additional ligation ring
620b, 720b, 820b Inner gap
620A, 720A, 820A First projection
740A, 740A, 840A Second projection
940A, 1040A, 1140A Third projection
930A, 1030A, 1130A Fourth projection
1320A Fifth projection
1240A Sixth projection
1340A Seventh projection
1230A Eighth projection
720c First groove
840b Second groove
1040b Third groove
1130b Fourth groove

## Claims

1. A ligation device comprising:
a cylindrical inner cylinder having a distal end portion ;
a slider having a cylindrical shape, attached around the inner cylinder, movable with respect to the inner cylinder along an axial direction of the inner cylinder, and having an annular groove extending along a circumferential direction on an inner peripheral face, and
a ligation ring attached around the inner cylinder and located in the annular groove.

2. The ligation device according to claim 1, wherein the slider is configured to be movable along the axial direction by a fluid.

3. The ligation device according to claim 1 or 2, wherein the slider is configured to be reciprocable along the axial direction.

4. The ligation device according to any one of claims 1 to 3, wherein the slider is composed of a plurality of slider pieces divided together with the annular groove.

5. The ligation device according to claim 4, wherein the slider is divided into the plurality of slider pieces along a plane comprising an axis of the slider or a plane perpendicular to the axis.

6. The ligation device according to any one of claims 1 to 5, wherein the distal end portion of the inner cylinder comprises a tapered portion having an outer diameter decreasing toward the distal end.

7. The ligation device according to claim 6, wherein a length of the tapered portion is larger than a distance from a distal end of the slider to a proximal end of the annular groove.

8. The ligation device according to claim 6 or 7, wherein a difference between an outer diameter at a proximal end of the tapered portion and an outer diameter at a distal end of the tapered portion is larger than a difference between an outer diameter and an inner diameter of the ligation ring.

9. The ligation device according to any one of claims 1 to 8, wherein a part of the ligation ring is temporarily fixed to a bottom face of the annular groove, and an inner peripheral side of the part is in non-contact with an outer peripheral face of the inner cylinder.

10. The ligation device according to any one of claims 1 to 9, wherein
a plurality of the annular grooves are formed on the inner peripheral face of the slider, and
a plurality of the ligation rings are attached to the outer peripheral face of the inner cylinder such that the plurality of ligation rings correspond to the plurality of annular grooves.

11. The ligation device according to any one of claims 1 to 10, wherein the inner cylinder is attachable around an endoscope distal end portion in an airtight or a liquidtight state.

12. The ligation device according to claim 11, wherein the slider is attached around the inner cylinder in an airtight or a liquidtight state.

13. The ligation device according to any one of claims 1 to 12, wherein a gap between the slider and the inner cylinder at a distal end portion and/or proximal end portion of the slider is smaller than a thickness of the ligation ring.

14. The ligation device according to any one of claims 1 to 13, further comprising an additional ligation ring located on the distal end side of the distal end of the slider and attached around the inner cylinder.

15. The ligation device according to claim 14, wherein a slider extension portion projecting toward an outer peripheral side of the additional ligation ring is placed on the distal end of the slider.

16. The ligation device according to any one of claims 1 to 15, wherein
an inner gap is formed between the inner cylinder and the slider,
a first projection that projects into the inner gap is placed on the outer peripheral face of the inner cylinder,
a second projection that projects into the inner gap is placed on an inner peripheral face of the slider, and
a sum of heights of the first projection and the second projection in a radial direction of the slider is structurally larger than a width of the inner gap in the radial direction.

17. The ligation device according to claim 16, wherein
a plurality of the first projections are placed along the axial direction, and
the radial-direction height of one of the plurality of first projections is different from the radial-direction heights of the other remaining first projections; or
a plurality of the second projections are placed along the axial direction, and
the radial-direction height of one of the plurality of second projections is different from the radial-direction heights of the other remaining second projections.

18. The ligation device according to claim 17, wherein
the plurality of first projections are configured such that their radial-direction heights gradually increase toward the distal end side in the axial direction, or
the plurality of second projections are configured such that their radial-direction heights gradually increase toward the proximal end side in the axial direction.

19. The ligation device according to any one of claims 16 to 18, wherein
a first groove extending along the axial direction is formed on the outer peripheral face of the inner cylinder,
the first projections project from a bottom portion of the first groove toward the inner gap, and
top portions of the second projections are accommodated in the first groove; and/or
a second groove extending along the axial direction is formed on an inner peripheral face of the slider,
the second projections project from a bottom portion of the second groove toward the inner gap, and
top portions of the first projections are accommodated in the second groove.

20. The ligation device according to any one of claims 1 to 19, further comprising an outer cylinder fixed to the inner cylinder and placed around the slider.

21. The ligation device according to claim 19, wherein
an outer gap is formed between the slider and the outer cylinder,
a third projection that projects into the outer gap is placed on an outer peripheral face of the slider,
a fourth projection that projects into the outer gap is placed on an inner peripheral face of the outer cylinder, and
a sum of heights of the third projection and the fourth projection in the radial direction of the slider is structurally larger than a width of the outer gap in the radial direction.

22. The ligation device according to claim 21, wherein
a plurality of the third projections are placed along the axial direction, and
the radial-direction height of one of the plurality of third projections is different from the radial-direction heights of the other remaining third projections; or
a plurality of the fourth projections are placed along the axial direction, and
the radial-direction height of one of the plurality of fourth projections is different from the radial-direction heights of the other remaining fourth projections.

23. The ligation device according to claim 22, wherein
the plurality of third projections are configured such that their radial-direction heights gradually increase toward the distal end side in the axial direction, or
the plurality of fourth projections are configured such that their radial-direction heights gradually increase toward the distal end side in the axial direction.

24. The ligation device according to any one of claims 21 to 23, wherein
a third groove extending along the axial direction is formed on the outer peripheral face of the slider,
the third projections project from a bottom portion of the third groove toward the outer gap, and
top portions of the fourth projections are accommodated in the thirteenth; and/or
a fourth groove extending along the axial direction is formed on the inner peripheral face of the outer cylinder,
the fourth projections project from a bottom portion of the fourth groove toward the outer gap, and
top portions of the third projections are accommodated in the fourth groove.

25. The ligation device according to any one of claims 1 to 24, wherein
fifth projections that project toward the inner peripheral face of the slider to abut on the inner peripheral face of the slider are placed on the outer peripheral face of the inner cylinder, and/or
sixth projections that project toward the outer peripheral face of the inner cylinder to abut on the outer peripheral face of the inner cylinder are placed on the inner peripheral face of the slider.

26. The ligation device according to claim 25, wherein
the fifth projections and the sixth projections extend along the axial direction.

27. The ligation device according to any one of claims 20 to 26, wherein
seventh projections that project toward the inner peripheral face of the outer cylinder to abut on the inner peripheral face of the outer cylinder are placed on the outer peripheral face of the slider, and/or
eighth projections that project toward the outer peripheral face of the slider to abut on the outer peripheral face of the slider are placed on the inner peripheral face of the outer cylinder.

28. The ligation device according to claim 27, wherein the seventh projections and the eighth projections extend along the axial direction.

29. The ligation device according to any one of claims 1 to 28, wherein a projection on which a distal end of the endoscope distal end portion is abuttable is placed on the inner peripheral face of the inner cylinder.

30. The ligation device according to claim 29, wherein the ligation ring is disposed at a position corresponding to the projection or a position opposite to the distal end portion side of the inner cylinder with respect to the projection in a state that the slider is disposed at a position opposite to the distal end portion side of the inner cylinder.
